# EUROPEAN PATENT APPLICATION

(11) **EP 2 052 738 A1**
(43) Date of publication of application: **29.04.2009**
(21) Application number: 07792417.3
(22) Date of filing: 10.08.2007
(51) Int. Cl.: A61K 45/00, A61K 38/22, A61P 19/08, A61P 25/00, A61P 43/00

(54) **THERAPEUTIC AGENT FOR ACCELERATION OF SPINAL NERVE REPAIR COMPRISING GHRELIN, DERIVATIVE THEREOF OR SUBSTANCE CAPABLE OF ACTING ON GHS-R1a AS ACTIVE INGREDIENT**

(30) Priority: 11.08.2006 JP 2006220573
(71) Applicant: University of Miyazaki, Miyazaki-shi Miyazaki 889-2192 (JP); Asubio Pharma Co., Ltd., Minato-ku Tokyo 107-8541 (JP); Japan as represented by president of National Cardiovascular Center, Suita-shi, Osaka 565-8565 (JP)
(72) Inventor: MURAKAMI, Noboru, Miyazaki-shi, Miyazaki 8800033 (JP); NAKAHARA, Keiko, Miyazaki-gun, Miyazaki 8891601 (JP); HASHIMOTO, Miho, Maebashi-shi, Gunma 3710037 (JP); HAYASHI, Yujiro, Ohra-gun, Gunma 3700503 (JP); KANGAWA, Kenji, Suita-shi, Osaka 5658565 (JP)
(74) Representative: Vossius & Partner
(86) International application number: PCT/JP2007/065774
(87) International publication number: WO 2008/018600

(57) **Abstract**

The invention provides a spinal neuron damage treating agent for use in the treatment of spinal neuron damage, or an agent for promoting the proliferation of spinal neuronal precursor cells in the culture of spinal neuronal precursor cells, or an agent for promoting the regeneration of spinal nerves after transplantation of cultured spinal neuronal precursor cells, and the like.

The invention provides an agent that contains a substance (e.g., ghrelin) that acts on the growth hormone secretagogue-receptor as an active ingredient, the agent being a spinal neuron damage treating agent for use in the treatment of spinal neuron damage, or an agent for promoting the proliferation of cultured spinal neuronal precursor cells in the culture of spinal neuronal precursor cells, or an agent for promoting the regeneration of spinal nerves after transplantation of cultured spinal neuronal precursor cells, and the like.

<110> University of Miyazaki,
      Asubio Pharma.Co.Ltd.,
      Japan as represented by President of National Cardiovascular Center
<120> SPINAL NERVE REPAIR PROMOTING THERAPEUTICS CONTAINING GHRELIN OR ITS DERIVATIVES OR SUBSTANCES THAT
ACT ON GHS-R1a AS AN ACTIVE INGREDIENT
<130> FA0011-07112
<150> JP 2006-220573
<151> 2006-8-11
<160> 25

<210>  1
<211>  28
<212>  PRT
<213>  Homo sapiens
<221>  PEPTIDE
<223>  Amino acid sequence for human endogenous peptides of growth hormo
       ne secretagogue
<400>  1
Gly Ser Ser Phe Leu Ser Pro Glu His Gln Arg Val Gln Gln Arg Lys
1               5                   10                  15
Glu Ser Lys Lys Pro Pro Ala Lys Leu Gln Pro Arg
            20                  25

<210>  2
<211>  27
<212>  PRT
<213>  Homo sapiens
<220>
<221>  PEPTIDE
<223>  Amino acid sequence for human endogenous peptides (27 amino acids
       ) of growth hormone secretagogue
<400>  2
Gly Ser Ser Phe Leu Ser Pro Glu His Gln Arg Val Gln Arg Lys Glu
1               5                   10                  15
Ser Lys Lys Pro Pro Ala Lys Leu Gln Pro Arg
            20                  25

<210>  3
<211>  28
<212>  PRT
<213>  Rattus norvegicus
<221>  PEPTIDE
<223>  Amino acid sequence for rat endogenous peptides of growth hormone
        secretagogue
<400>  3
Gly Ser Ser Phe Leu Ser Pro Glu His Gln Lys Ala Gln Gln Arg Lys
1               5                   10                  15
Glu Ser Lys Lys Pro Pro Ala Lys Leu Gln Pro Arg
            20                  25

<210>  4
<211>  27
<212>  PRT
<213>  Rattus norvegicus
<221>  PEPTIDE
<223>  Amiino acid sequence for rat endogenous peptides of growth hormon
       e secretagogue
<400>  4
Gly Ser Ser Phe Leu Ser Pro Glu His Gln Lys Ala Gln Arg Lys Glu
1               5                   10                  15
Ser Lys Lys Pro Pro Ala Lys Leu Gln Pro Arg
            20                  25

<210>  5
<211>  28
<212>  PRT
<213>  Mus musculus
<221>  PEPTIDE
<223>  Amino acid sequence for mouse endogenous peptides of growth hormo
       ne secretagogue
<400>  5
Gly Ser Ser Phe Leu Ser Pro Glu His Gln Lys Ala Gln Gln Arg Lys
1               5                   10                  15
Glu Ser Lys Lys Pro Pro Ala Lys Leu Gln Pro Arg
            20                  25

<210> 6
<211> 28
<212> PRT
<213> Sus scrofa
<221> PEPTIDE
<223> Amino acid sequence for porcine endogenous peptides of growth hor
      mone secretagogue
<400> 6
Gly Ser Ser Phe Leu Ser Pro Glu His Gln Lys Val Gln Gln Arg Lys
1               5                   10                  15
Glu Ser Lys Lys Pro Ala Ala Lys Leu Lys Pro Arg
            20                  25

<210> 7
<211> 27
<212> PRT
<213> Bos taurus
<221> PEPTIDE
<223> Amino acid sequence for bovine endogenous peptides (27 amino acid
      s) of growth hormone secretagogue
<400> 7
Gly Ser Ser Phe Leu Ser Pro Glu His Gln Lys Leu Gln Arg Lys Glu
1               5                   10                  15
Ala Lys Lys Pro Ser Gly Arg Leu Lys Pro Arg
            20                  25

<210> 8
<211> 27
<212> PRT
<213> Ovis aries
<221> PEPTIDE
<223> Amino acid sequence for ovine endogenous peptides (27 amino acids
      ) of growth hormone secretagogue
<400> 8
Gly Ser Ser Phe Leu Ser Pro Glu His Gln Lys Leu Gln Arg Lys Glu
1               5                   10                  15
Pro Lys Lys Pro Ser Gly Arg Leu Lys Pro Arg
            20                  25

<210> 9
<211> 28
<212> PRT
<213> Canis familiaris
<221> PEPTIDE
<223> Amino acid sequence for dog endogenous peptides of growth hormone
       secretagogue
<400> 9
Gly Ser Ser Phe Leu Ser Pro Glu His Gln Lys Leu Gln Gln Arg Lys
1               5                   10                  15
Glu Ser Lys Lys Pro Pro Ala Lys Leu Gln Pro Arg
            20                  25

<210> 10
<211> 21
<212> PRT
<213> Anguilla japonica
<221> PEPTIDE
<223> Amino acid sequence for eel endogenous peptides of growth hormone
       secretagogue. This peptide is amidated at C-terminus.
<400> 10
Gly Ser Ser Phe Leu Ser Pro Ser Gln Arg Pro Gln Gly Lys Asp Lys
1               5                   10                  15
Lys Pro Pro Arg Val
            20

<210> 11
<211> 23
<212> PRT
<213> Oncorhynchus mykiss
<221> PEPTIDE
<223> Amino acid sequence for rainbow trout endogenous peptides (23 ami
      no acids) of growth hormone secretagogue. This peptide is amidate
      d at C-terminus.
<400> 11
Gly Ser Ser Phe Leu Ser Pro Ser Gln Lys Pro Gln Val Arg Gln Gly
1               5                   10                  15

Lys Gly Lys Pro Pro Arg Val
            20

<210>  12
<211>  20
<212>  PRT
<213>  Oncorhynchus mykiss
<221>  PEPTIDE
<223>  Amino acid sequence for rainbow trout endogenous peptides (20 ami
       no acids) of growth hormone secretagogue.  This peptide is amidat
       ed at C-terminus.
<400>  12
Gly Ser Ser Phe Leu Ser Pro Ser Gln Lys Pro Gln Gly Lys Gly Lys
1               5                   10                  15
Pro Pro Arg Val
            20

<210>  13
<211>  24
<212>  PRT
<213>  Gallus domesticus
<221>  PEPTIDE
<223>  Amino acid sequence for chicken endogenous peptides of growth hor
       mone secretagogue
<400>  13
Gly Ser Ser Phe Leu Ser Pro Thr Tyr Lys Asn Ile Gln Gln Gln Lys
1               5                   10                  15
Gly Thr Arg Lys Pro Thr Ala Arg
            20

<210>  14
<211>  24
<212>  PRT
<213>  Gallus domesticus
<221>  PEPTIDE
<223>  Amino acid sequence for chicken endogenous peptides of growth hor
       mone secretagogue
<400>  14
Gly Ser Ser Phe Leu Ser Pro Thr Tyr Lys Asn Ile Gln Gln Gln Lys
1               5                   10                  15
Asp Thr Arg Lys Pro Thr Ala Arg
            20

<210>  15
<211>  26
<212>  PRT
<213>  Gallus domesticus
<221>  PEPTIDE
<223>  Amino acid sequence for chicken endogenous peptides of growth hor
       mone secretagogue
<400>  15
Gly Ser Ser Phe Leu Ser Pro Thr Tyr Lys Asn Ile Gln Gln Gln Lys
1               5                   10                  15
Asp Thr Arg Lys Pro Thr Ala Arg Leu His
            20                  25

<210>  16
<211>  27
<212>  PRT
<213>  Rana catesbeiana
<221>  PEPTIDE
<223>  Amino acid sequence for frog endogenous peptides of growth hormon
       e secretagogue
<400>  16
Gly Leu Thr Phe Leu Ser Pro Ala Asp Met Gln Lys Ile Ala Glu Arg
1               5                   10                  15
Gln Ser Gln Asn Lys Leu Arg His Gly Asn Met
            20                  25

<210>  17
<211>  28
<212>  PRT
<213>  Rana catesbeiana
<221>  PEPTIDE
<223>  Amino acid sequence for frog endogenous peptides of growth hormon
       e secretagogue

<400> 17
Gly Leu Thr Phe Leu Ser Pro Ala Asp Met Gln Lys Ile Ala Glu Arg
1               5                   10                  15
Gln Ser Gln Asn Lys Leu Arg His Gly Asn Met Asn
            20                  25

<210> 18
<211> 20
<212> PRT
<213> Tilapia nilotica
<221> PEPTIDE
<223> Amino acid sequence for tilapia endogenous peptides of growth hor
      mone secretagogue. This peptide is amidated at C-terminus.
<400> 18
Gly Ser Ser Phe Leu Ser Pro Ser Gln Lys Pro Gln Asn Lys Val Lys
1               5                   10                  15
Ser Ser Arg Ile
            20

<210> 19
<211> 22
<212> PRT
<213> Silurus asotus
<221> PEPTIDE
<223> Amino acid sequence for catfish endogenous peptides of growth hor
      mone secretagogue. This peptide is amidated at C-terminus.
<400> 19
Gly Ser Ser Phe Leu Ser Pro Thr Gln Lys Pro Gln Asn Arg Gly Asp
1               5                   10                  15
Arg Lys Pro Pro Arg Val
            20

<210> 20
<211> 23
<212> PRT
<213> Silurus asotus
<221> PEPTIDE
<223> Amino acid sequence for catfish endogenous peptides of growth hor
      mone secretagogue
<400> 20
Gly Ser Ser Phe Leu Ser Pro Thr Gln Lys Pro Gln Asn Arg Gly Asp
1               5                   10                  15
Arg Lys Pro Pro Arg Val Gly
            20

<210> 21
<211> 28
<212> PRT
<213> Equus caballus
<221> PEPTIDE
<223> Amino acid sequence for equine endogenous peptides of growth horm
      one secretagogue
<400> 21
Gly Ser Ser Phe Leu Ser Pro Glu His His Lys Val Gln His Arg Lys
1               5                   10                  15
Glu Ser Lys Lys Pro Pro Ala Lys Leu Lys Pro Arg
            20                  25

<210> 22
<211> 28
<212> DNA
<213> Artificial Sequence
<220>
<223> Primer (GHS-R 1a), sense
<400> 22
gatacctctt ttccaagtcc ttcgagcc                                    28

<210> 23
<211> 25
<212> DNA
<213> Artificial Sequence
<220>
<223> Primer (GHS-R 1a), antisense
<400> 23
ttgaacactg ccacccggta cttct                                       25

<210> 24
<211> 19
<212> DNA
<213> Artificial Sequence
<220>
<223> Primer (GAPDH), sense
<400> 24
cggcaagttc aacggcaca 19

<210> 25
<211> 23
<212> DNA
<213> Artificial Sequence
<220>
<223> Primer (GAPDH), antisense
<400> 25
agacgccagt agactccacg aca 23

## Description

### TECHNICAL FIELD

The present invention relates to the spinal neuronal precursor cell proliferating action of substances that act on the growth hormone secretagogue receptor. More particularly, the invention relates to agents that contain those substances as an active ingredient, such as spinal nerve damage treating agents for use in the treatment of spinal nerve damage, agents for promoting the proliferation of spinal neuronal precursor cells in the culture of spinal neuronal precursor cells, and agents for promoting the regeneration of spinal nerves after transplantation of cultured spinal neuronal precursor cells.

### BACKGROUND ART

More than 6000 people suffer from spinal cord injury annually as the result of traffic accidents, sport accidents and occupational accidents, with a total of about 110,000 victims being counted across the nation. This is estimated to cost a social loss of as much as 300 billion yen per year and it has been said that there is no method of treatment (Non-Patent Document 1). Spinal nerves have so far been considered to be non-regenerable or non-transplantable; however, as a tissue, the transplantation of spinal nerves is recently being investigated at an experimental level as in the case of fetal spinal cord cells and there is suggested possibility for their treatment by transplantation. Furthermore, in view of the future research on transplantation and regeneration, spinal nerves may be considered to be a tissue the development and regeneration processes of which are anticipated to be verified in the years to come by means of such studies as the research on the development of spinal nerves and regeneration research using cultured fetal spinal neurons. In addition, the research on the transplantation and regeneration of spinal nerves is anticipated to play an important role in spinal cord injury, spinal cord tumor, brain tumor or cranial nerve disorder.

As a matter of fact, the preclinical research on nerve regeneration is making steady advances. Cells and substances that have the potential to repair and regenerate nerves include human stem cells, nasal mucosal cells, myeloid series of cells such as stromal marrow cells, umbilical blood, macrophages, 4-aminopyridine (which is under large-scale phase III clinical trial), etc. Thus, a series of research reports have been made in the last two or three years about new cells and substances that show the potential for nerve repair and regeneration. Some of these studies are in the process of transition from the level of animal experiment to the clinical study in humans and in order to ensure that they will continue to develop constantly, the establishment of a medical center for spinal cord regeneration that is equipped with an integrated research system combining the preclinical with the clinical research is becoming an urgent goal in Japan (Non-Patent Document 2).

Transplantation of spinal cord cells is currently studied with animals at an experimental level and clinical research has begun in order to apply it to the treatment of spinal cord injuries in humans. Cells that are used in preclinical or clinical research include human stem cells, nasal mucosal cells, myeloid series of cells, umbilical blood, macrophages, and tissues from embryos (Non-Patent Document 2).

Another source that is used in the research on nerve regeneration is tissues from embryos. In animal experiments, they drew attention as a source that would show high capability for regeneration and the nerve stem cells prepared from a monkey fetal spinal cord were transplanted in a monkey with a damaged spinal cord, which successfully recovered its function (Non-Patent Document 3); however, fetal cells generally proliferate so slowly that development of a proliferation promoting technique is desired.

Further, the clinical application of GM-CSF, lectin and the like that are used in neuronal regenerative therapy involves concern about cell differentiation at other sites in the human body and side-effects and, hence, it is desired to discover and develop substances that are highly safe while having the intended activity (Non-Patent Document 4).

Ghrelin, a hormone discovered from the stomach in 1999, is a peptide having an amino acid sequence composed of 28 residues and it has a quite extraordinary chemical structure in that the third amino acid from the amino terminal of the sequence is acylated with a fatty acid (Non-Patent Document 5 and Patent Document 1). Ghrelin is an endogenous brain-gut hormone that acts as a growth hormone secretagogue-receptor 1a (GHS-R1a) (Non-Patent Document 6) to stimulate the secretion of growth hormone (GH) from the pituitary (Non-Patent 5).

In addition, ghrelin was first isolated and purified from the rat as an endogenous GHS for GHS-R1a and vertebral animals other than rat, such as human, mouse, porcine, chicken, eel, bovine, equine, ovine, frog, trout, and canine, are known to have amino acid sequences of ghrelin having similar primary structures (Patent Document 1).
Human
   :GSS(n-octanoyl)FLSPEHQRVQQRKESKKPPAKLQPR (corresponding to SEQ ID NO: 1)
   :GSS(n-octanoyl)FLSPEHQRVQRKESKKPPAKLQPR (corresponding to SEQ ID NO:2)
Rat
   :GSS(n-octanoyl)FLSPEHQKAQQRKESKKPPAKLQPR (corresponding to SEQ ID NO:3)
   :GSS(n-octanoyl)FLSPEHQKAQRKESKKPPAKLQPR (corresponding to SEQ ID NO:4)
Mouse
   :GSS(n-octanoyl)FLSPEHQKAQQRKESKKPPAKLQPR (corresponding to SEQ ID NO:5)
Porcine
   :GSS(n-octanoyl)FLSPEHQKVQQRKESKKPAAKLKPR (corresponding to SEQ ID NO:6)
Bovine
   :GSS(n-octanoyl)FLSPEHQKLQRKEAKKPSGRLKPR (corresponding to SEQ ID NO:7)
Ovine
   : GSS(n-octanoyl)FLSPEHQKLQRKEPKKPSGRLKPR (corresponding to SEQ ID NO:8)
Canine
   : GSS(n-octanoyl)FLSPEHQKLQQRKESKKPPAKLOPR (corresponding to SEQ ID NO:9)
Eel
   :GSS(n-octanoyl)FLSPSQRPQGKDKKPPRV-NH₂ (corresponding to SEQ ID NO:10)
Trout
   : GSS(n-octanoyl)FLSPSQKPQVRQGKGKPPRV-NH₂ (corresponding to SEQ ID NO:11)
   : GSS(n-octanoyl)FLSPSQKPQGKGKPPRV-NH₂ (corresponding to SEQ ID NO:12)
Chicken
   : GSS(n-octanoyl)FLSPTYKNIQQQKGTRKPTAR (corresponding to SEQ ID NO:13)
   : GSS(n-octanoyl)FLSPTYKNIQOOKOTRKPTAR (corresponding to SEQ ID NO:14)
   : GSS(n-octanoyl)FLSPTYKNIQQQKDTRKPTARLH (corresponding to SEQ ID NO:15)
Bullfrog
   : GLT(n-octanoyl)FLSPADMQKIAERQSQNKLRHGNM (corresponding to SEQ ID NO:16)
   : GLT(n-decanoyl)FLSPADMQKIAERQSQNKLRHGNM (corresponding to SEQ ID NO:16)
   : GLT(n-octanoyl)FLSPADMQKIAERQSQNKLRHGNMN (corresponding to SEQ ID NO:17)
Tilapia
   : GSS(n-octanoyl)FLSPSQKPONKVKSSRI-NH₂ (corresponding to SEQ ID NO:18)
Catfish
   : GSS(n-octanoyl)FLSPTQKPQNRGDRKPPRV-NH₂ (corresponding to SEQ ID NO:19)
   : GSS(n-octanoyl)FLSPTQKPQNRGDRKPPRVG (corresponding to SEQ ID NO:20)
Equine
   : GSS(n-butanoyl)FLSPEHHKVQHRKESKKPPAKLKPRb(corresponding to SEQ ID NO:21)
(In the above designations, amino acid residues are represented by the single-letter code.)

The above-mentioned peptides are those having such a unique structure that the side-chain hydroxyl group in the serine residue (S) or threonine residue (T) at position 3 is acylated with a fatty acid such as octanoic acid or decanoic acid and no physiologically active peptides other than ghrelin that have such a hydrophobic, modified structure have been isolated from the living body.

Aside from the above-mentioned compounds, there are other substances that act on GHS-R1a and they include GHRP-2 which is also a peptide compound and MK-0677 which is a low-molecular weight compound.

Recent studies have revealed that ghrelin enhances appetite and that, when administered subcutaneously, it increases body weight and body fat (Non-Patent Documents 7 to 9), as well as having such actions as an improvement in cardiac function (Non-Patent Documents 10 to 12).

Further, with ghrelin having a GH secretion stimulating action and an appetite enhancing action, it is anticipated that the action of burning the fat and converting it to energy via the action of GH, or alternatively, the effect of increasing the muscular strength by developing the anabolic action of GH, can be elicited more effectively by enhanced appetite (Non-Patent Document 13).

The present inventors found that ghrelin, when administered to a pregnant mother animal (rat), promoted the growth of the embryos and that the administered substance transferred to the amniotic fluid as well as to the embryos; upon making a study considering the functions and roles of substances that would act on the growth hormone secretagogue-receptor (GHS-R1a) in the amniotic fluid, they found that GHS-R1a was present on the skin cells of embryos and that ghrelin had the action of proliferating the fetal skin cells (Non-Patent Document 14).

It is known that ghrelin has a neurite elongating action in a line of rat adrenal brown cytoma cell PC12 (Patent Document 2). PC12 cells are widely used as a neuron model in the study of a mechanism for a variety of neuron agonists such as a nerve growth factor (NGF). However, PC12 cells are tumor cells derived from adrenal brown cytoma; since they are tumor cells that express a great number of active substances and receptors, including neurotransmitters and physiologically active peptides such as catecholamine and PACAP (pituitary acenylate cyclase activating peptide) and adenosine receptors which are a variety of bioactive substances, research on their physiological actions requires studies under conditions even more similar to physiological conditions as in the primary culture system, or *in vivo* or *extra vivo* experiments. It has also been reported that ghrelin shows a neuron proliferating action on the dorsal motor nucleus of the vagus (DMNV) and on nuclei of solitary tract by elevating the intracellular calcium concentration via GHS-R1a (Non-Patent Documents 15 and 16). However, no observation has been made about the action of those substances (e.g. ghrelin) which act on GHS-R1a for fetal spinal neuronal precursor cells that, when transplanted in the central nervous system, will cause neurons to grow to thereby prove promising in the treatment of spinal cord injury, and further advances in research are required to develop a technique directed toward regeneration and transplantation of spinal neurons. Further in addition, the present inventors found in the present invention that the L-type calcium channel blocker diltiazem did not suppress the spinal neuron proliferating action of ghrelin and from this finding, it is believed that the nerve proliferating action in DMNV is something that is different from the spinal neuron proliferating action.
[Patent Document 1] WO 01/07475
[Patent Document 2] JP 2005-239712 A
[Non-Patent Document 1] Iryo In Focus (Medicine in Focus), Part II, Saisei Iryo (Regenerative Medicine), 6 "Sekizui Shinkei" Saisei (Regeneration of Spinal Cord Nerves), May 21, 2005
   (URL:http://www.ubenippo.co.jp/infocus/saisei/saisei 6.html)
[Non-Patent Document 2] Shinkeisaiseikenkyu ni okeru taijisoshikiriyo ni kannsuru kenkai (Opinion Concerning the Utilization of Fetal Tissues in the Research on Nerve Regeneration), April 5, 2005, Japan Spinal Cord Foundation, an incorporated non-profit organization
   (URL : http://www.jscf.org/jscf/SIRYOU/igaku/-1/saiboisyoku/jscf050405.html)
[Non-Patent Document 3] Shinkeikansaibo de kotsuzuisonshokaifuku (Recovery from Spinal Cord Injury Using Neural Stem Cells), Article from December 10, 2001 issue of Tokyo Shinbun (URL : http:www.normanet.ne.jp/∼JSCF/SIRYOU/tokyo-2.htm)
[Non-Patent Document 4] Shinkeisaiseichiryo (Regeneration of Central Nerves System) (URL : http://www.ins-gbs.co.jp/nerve.html)
[Non-Patent Document 5] Kojima et al.: Nature, 402, pp. 656-660 (1999)
[Non-Patent Document 6] Howard et al. : Science, 273, pp. 974-977 (1996)
[Non-Patent Document 7] Wren et al.: Endocrinology, 141, pp. 4325-4328 (2000)
[Non-Patent Document 8] Nakazato et al.: Nature, 409, 194-198 (2001)
[Non-Patent Document 9] Shintani et al.: Diabetes, 50, pp. 227-232 (2001)
[Non-Patent Document 10] Nakazato et al.: Nature, 409, pp. 194-198 (2001)
[Non-Patent Document 11] Lely et al.: Endocr. Rev., 25, pp. 656-660 (2004)
[Non-Patent Document 12] Korbonits et al.: Front Neuroendocrinol., 25, pp. 27-68 (2004)
[Non-Patent Document 13] Kangawa et al.: J. Pharmacol. Sci., 100, pp. 398-410 (2006)
[Non-Patent Document 14] Nakahara et al.: Endocrinology, 147, pp. 1333-1342 (2006)
[Non-Patent Document 15] Zhang et al.: J Physiol., 559, pp. 729-737 (2004)
[Non-Patent Document 16] Zhang et al.: Peptides, 26, pp. 2280-2288 (2005)

### DISCLOSURE OF THE INVENTION

### PROBLEMS TO BE SOLVED BY THE INVENTION

The present invention relates to providing agents that employ substances having an action for proliferating spinal neuronal precursor cells, such as spinal neuron damage treating agents for use in the treatment of spinal neuron damage, agents for promoting the proliferation of spinal neuronal precursor cells in the culture of spinal neuronal precursor cells, and agents for promoting the regeneration of spinal nerves after transplantation of cultured spinal neuronal precursor cells.

### MEANS FOR SOLVING THE PROBLEMS

As described above, the present inventors found that when ghrelin, a substance acting on a growth hormone secretagogue-receptor, was administered to a pregnant mother animal (rat), it promoted the growth of the embryos and that the administered substance transferred to the amniotic fluid as well as to the embryos; they also found that the growth hormone secretagogue-receptor (GHS-R1a) was present on the skin cells of the embryos and that ghrelin had the action of proliferating the fetal skin cells.

Further, with a view to studying the action of the substance acting on the growth hormone secretagogue-receptor in embryos (rat), the present inventors searched for the site of presence of the growth hormone secretagogue-receptor by the RT-PCR technique and found that the growth hormone secretagogue-receptor was present in spinal neuronal precursor cells. The substance acting on the growth hormone secretagogue-receptor was then caused to act on spinal neuronal precursor cells, whereupon the present inventors found that the incorporation of BrdU was promoted and that the substance at issue showed the action of proliferating spinal neuronal precursor cells. Furthermore, in a test with rat models suffering a spinal cord injury, ghrelin was locally administered in combination with transplantation of cultured spinal neuronal precursor cells, whereupon the inventors confirmed a recovery of the impaired lower limb's function from the spinal cord injury. The present inventors had already found the expression of the growth hormone secretagogue-receptor in skin cells (Non-Patent Document 14) and in the present invention, they further found that the growth hormone secretagogue-receptor was also expressed in spinal neuronal precursor cells, which led to the finding that ghrelin, a substance acting on the growth hormone secretagogue-receptor, and its derivatives would act on spinal neuronal precursor cells to proliferate those cells, thus proving to be applicable to neuronal regenerative medicine.

Thus, the present invention relates to an agent that contains a substance that acts on the growth hormone secretagogue-receptor or a pharmaceutically acceptable salt thereof as an active ingredient, the agent being a spinal neuron damage treating agent for use in the treatment of spinal neuron damage, or an agent for promoting the proliferation of cultured spinal neuronal precursor cells in the culture of spinal neuronal precursor cells, or an agent for promoting the regeneration of spinal nerves after transplantation of cultured spinal neuronal precursor cells.

The present invention also relates to a method that comprises administering a substance that acts on the growth hormone secretagogue-receptor or a pharmaceutically acceptable salt thereof, the method being a method for treating spinal neuron damage in an individual, or a method for promoting the proliferation of cultured spinal neuronal precursor cells in the culture of spinal neuronal precursor cells, or a method for promoting the regeneration of spinal nerves after transplantation of cultured spinal neuronal precursor cells in an individual.

Further, the present invention relates to the use of a substance that acts on the growth hormone secretagogue-receptor or a pharmaceutically acceptable salt thereof for producing a spinal neuron damage treating agent for use in the treatment of spinal neuron damage, or an agent for promoting the proliferation of cultured spinal neuronal precursor cells in the culture of spinal neuronal precursor cells, or an agent for promoting the regeneration of spinal nerves after transplantation of cultured spinal neuronal precursor cells.

In view of the foregoing, the present invention specifically relates to the following items:
[1] A spinal neuron damage treating agent containing a substance that acts on a growth hormone secretagogue-receptor or a pharmaceutically acceptable salt thereof as an active ingredient.
[2] The treating agent of [1] above, wherein the substance is a peptide selected from the group consisting of:
   (1) a peptide whose amino acid sequence is represented by any one of SEQ ID NO:1 to SEQ ID NO:21, provided that the third amino acid residue from the amino terminus is a modified amino acid residue having a fatty acid introduced in the side chain of that amino acid residue;
   (2) a peptide whose amino acid sequence is represented by any one of SEQ ID NO:1 to SEQ ID NO:21, provided that one or several amino acids in the sequence of from the 5th up to the 28th amino acid from the amino terminus are deleted, substituted and/or added and that the third amino acid residue from the amino terminus is a modified amino acid residue having a fatty acid introduced in the side chain of that amino acid residue, the peptide having a spinal neuronal precursor cell proliferating action; and
   (3) a peptide having a sequence of up to at least the 4th amino acid from the amino terminus of the amino acid sequence that is represented by any one of SEQ ID NO:1 to SEQ ID NO:21, with the third amino acid residue from the amino terminus being a modified amino acid residue having a fatty acid introduced in the side chain of that amino acid residue, the peptide having a spinal neuronal precursor cell proliferating action,
   or a derivative thereof.
[3] The treating agent of [2] above, wherein the substance is a peptide whose amino acid sequence is represented by SEQ ID NO:1, provided that the serine residue which is positioned the third from the amino terminus is a modified amino acid residue having a fatty acid introduced in the hydroxyl group at the side chain of that residue.
[4] The treating agent of [3] above, wherein the substance is a peptide whose amino acid sequence is represented by SEQ ID NO:1, provided that the hydroxyl group at the side chain of the serine residue which is positioned the third from the amino terminus is acylated by an n-octanoyl group.
[5] The treating agent as recited in [1] to [4] above, which contains 0.001 mg to 100 mg of the substance or pharmaceutically acceptable salt thereof.
[6] An agent for promoting the proliferation of spinal neuronal precursor cells in the culture of spinal neuronal precursor cells that contains a substance that acts on a growth hormone secretagogue-receptor or a salt thereof as an active ingredient.
[7] The promoting agent of [6] above, wherein the substance is a peptide selected from the group consisting of:
   (1) a peptide whose amino acid sequence is represented by any one of SEQ ID NO:1 to SEQ ID NO:21, provided that the third amino acid residue from the amino terminus is a modified amino acid residue having a fatty acid introduced in the side chain of that amino acid residue;
   (2) a peptide whose amino acid sequence is represented by any one of SEQ ID NO:1 to SEQ ID NO:21, provided that one or several amino acids in the sequence of from the 5th up to the 28th amino acid from the amino terminus are deleted, substituted and/or added and that the third amino acid residue from the amino terminus is a modified amino acid residue having a fatty acid introduced in the side chain of that amino acid residue, the peptide having a spinal neuronal precursor cell proliferating action; and
   (3) a peptide having a sequence of up to at least the 4th amino acid from the amino terminus of the amino acid sequence that is represented by any one of SEQ ID NO:1 to SEQ ID NO:21, with the third amino acid residue from the amino terminus being a modified amino acid residue having a fatty acid introduced in the side chain of that amino acid residue, the peptide having a spinal neuronal precursor cell proliferating action,
   or a derivative thereof.
[8] The promoting agent of [7] above, wherein the substance is a peptide whose amino acid sequence is represented by SEQ ID NO:1, provided that the serine residue which is positioned the third from the amino terminus is a modified amino acid residue having a fatty acid introduced in the hydroxyl group at the side chain of that residue.
[9] The promoting agent of [8] above, wherein the substance is a peptide whose amino acid sequence is represented by SEQ ID NO:1, provided that the hydroxyl group at the side chain of the serine residue which is positioned the third from the amino terminus is acylated by an n-octanoyl group.
[10] The promoting agent as recited in [6] to [9] above, wherein the content of the substance or salt thereof in the culture medium for spinal neuronal precursor cells is from 0.0000001 mg/L to 0.1 mg/L.
[11] An agent for promoting the regeneration of spinal nerves after transplantation of cultured spinal neuronal precursor cells that contains a substance that acts on a growth hormone secretagogue-receptor or a pharmaceutically acceptable salt thereof as an active ingredient.
[12] The promoting agent of [11] above, wherein the substance is a peptide selected from the group consisting of:
   (1) a peptide whose amino acid sequence is represented by any one of SEQ ID NO:1 to SEQ ID NO:21, provided that the third amino acid residue from the amino terminus is a modified amino acid residue having a fatty acid introduced in the side chain of that amino acid residue;
   (2) a peptide whose amino acid sequence is represented by any one of SEQ ID NO:1 to SEQ ID NO:21, provided that one or several amino acids in the sequence of from the 5th up to the 28th amino acid from the amino terminus are deleted, substituted and/or added and that the third amino acid residue from the amino terminus is a modified amino acid residue having a fatty acid introduced in the side chain of that amino acid residue, the peptide having a spinal neuronal precursor cell proliferating action; and
   (3) a peptide having a sequence of up to at least the 4th amino acid from the amino terminus of the amino acid sequence that is represented by any one of SEQ ID NO:1 to SEQ ID NO:21, with the third amino acid residue from the amino terminus being a modified amino acid residue having a fatty acid introduced in the side chain of that amino acid residue, the peptide having a spinal neuronal precursor cell proliferating action,
   or a derivative thereof.
[13] The promoting agent of [12] above, wherein the substance is a peptide whose amino acid sequence is represented by SEQ ID NO:1, provided that the serine residue which is positioned the third from the amino terminus is a modified amino acid residue having a fatty acid introduced in the hydroxyl group at the side chain of that residue.
[14] The promoting agent of [13] above, wherein the substance is a peptide whose amino acid sequence is represented by SEQ ID NO:1, provided that the hydroxyl group at the side chain of the serine residue which is positioned the third from the amino terminus is acylated by an n-octanoyl group.
[15] The promoting agent as recited in [11] to [14] above, which contains 0.001 mg to 100 mg of the substance or pharmaceutically acceptable salt thereof.
[16] A method for treating spinal neuron damage that comprises administering an individual with a substance that acts on a growth hormone secretagogue-receptor or a pharmaceutically acceptable salt thereof.
[17] The method of [16] above, wherein the substance is a peptide selected from the group consisting of:
   (1) a peptide whose amino acid sequence is represented by any one of SEQ ID NO:1 to SEQ ID NO:21, provided that the third amino acid residue from the amino terminus is a modified amino acid residue having a fatty acid introduced in the side chain of that amino acid residue;
   (2) a peptide whose amino acid sequence is represented by any one of SEQ ID NO:1 to SEQ ID NO:21, provided that one or several amino acids in the sequence of from the 5th up to the 28th amino acid from the amino terminus are deleted, substituted and/or added and that the third amino acid residue from the amino terminus is a modified amino acid residue having a fatty acid introduced in the side chain of that amino acid residue, the peptide having a spinal neuronal precursor cell proliferating action; and
   (3) a peptide having a sequence of up to at least the 4th amino acid from the amino terminus of the amino acid sequence that is represented by any one of SEQ ID NO:1 to SEQ ID NO:21, with the third amino acid residue from the amino terminus being a modified amino acid residue having a fatty acid introduced in the side chain of that amino acid residue, the peptide having a spinal neuronal precursor cell proliferating action,
   or a derivative thereof.
[18] The method of [17] above, wherein the substance is a peptide whose amino acid sequence is represented by SEQ ID NO:1, provided that the serine residue which is positioned the third from the amino terminus is a modified amino acid residue having a fatty acid introduced in the hydroxyl group at the side chain of that residue.
[19] The method of [18] above, wherein the substance is a peptide whose amino acid sequence is represented by SEQ ID NO:1, provided that the hydroxyl group at the side chain of the serine residue which is positioned the third from the amino terminus is acylated by an n-octanoyl group.
[20] The method as recited in [16] to [19] above, which comprises administering 0.001 mg to 100 mg of the substance or pharmaceutically acceptable salt thereof.
[21] A method for promoting the proliferation of cultured spinal neuronal precursor cells, characterized by using a substance that acts on a growth hormone secretagogue-receptor or a salt thereof.
[22] The method of [21] above, wherein the substance is a peptide selected from the group consisting of:
   (1) a peptide whose amino acid sequence is represented by any one of SEQ ID NO:1 to SEQ ID NO:21, provided that the third amino acid residue from the amino terminus is a modified amino acid residue having a fatty acid introduced in the side chain of that amino acid residue;
   (2) a peptide whose amino acid sequence is represented by any one of SEQ ID NO:1 to SEQ ID NO:21, provided that one or several amino acids in the sequence of from the 5th up to the 28th amino acid from the amino terminus are deleted, substituted and/or added and that the third amino acid residue from the amino terminus is a modified amino acid residue having a fatty acid introduced in the side chain of that amino acid residue, the peptide having a spinal neuronal precursor cell proliferating action; and
   (3) a peptide having a sequence of up to at least the 4th amino acid from the amino terminus of the amino acid sequence that is represented by any one of SEQ ID NO:1 to SEQ ID NO:21, with the third amino acid residue from the amino terminus being a modified amino acid residue having a fatty acid introduced in the side chain of that amino acid residue, the peptide having a spinal neuronal precursor cell proliferating action,
   or a derivative thereof.
[23] The method of [22] above, wherein the substance is a peptide whose amino acid sequence is represented by SEQ ID NO:1, provided that the serine residue which is positioned the third from the amino terminus is a modified amino acid residue having a fatty acid introduced in the hydroxyl group at the side chain of that residue.
[24] The method of [23] above, wherein the substance is a peptide whose amino acid sequence is represented by SEQ ID NO:1, provided that the hydroxyl group at the side chain of the serine residue which is positioned the third from the amino terminus is acylated by an n-octanoyl group.
[25] The method as recited in [21] to [24] above, wherein the content of the substance or salt thereof in the culture medium for the cultured spinal neuronal precursor cells is from 0.0000001 mg/L to 0.1 mg/L.
[26] A method for promoting the regeneration of spinal nerves after transplantation of cultured spinal neuronal precursor cells, which comprises administering an individual with a substance that acts on a growth hormone secretagogue-receptor or a pharmaceutically acceptable salt thereof.
[27] The method of [26] above, wherein the substance is a peptide selected from the group consisting of:
   (1) a peptide whose amino acid sequence is represented by any one of SEQ ID NO:1 to SEQ ID NO:21, provided that the third amino acid residue from the amino terminus is a modified amino acid residue having a fatty acid introduced in the side chain of that amino acid residue;
   (2) a peptide whose amino acid sequence is represented by any one of SEQ ID NO:1 to SEQ ID NO:21, provided that one or several amino acids in the sequence of from the 5th up to the 28th amino acid from the amino terminus are deleted, substituted and/or added and that the third amino acid residue from the amino terminus is a modified amino acid residue having a fatty acid introduced in the side chain of that amino acid residue, the peptide having a spinal neuronal precursor cell proliferating action; and
   (3) a peptide having a sequence of up to at least the 4th amino acid from the amino terminus of the amino acid sequence that is represented by any one of SEQ ID NO:1 to SEQ ID NO:21, with the third amino acid residue from the amino terminus being a modified amino acid residue having a fatty acid introduced in the side chain of that amino acid residue, the peptide having a spinal neuronal precursor cell proliferating action,
   or a derivative thereof.
[28] The method of [27] above, wherein the substance is a peptide whose amino acid sequence is represented by SEQ ID NO:1, provided that the serine residue which is positioned the third from the amino terminus is a modified amino acid residue having a fatty acid introduced in the hydroxyl group at the side chain of that residue.
[29] The method of [28] above, wherein the substance is a peptide whose amino acid sequence is represented by SEQ ID NO:1, provided that the hydroxyl group at the side chain of the serine residue which is positioned the third from the amino terminus is acylated by an n-octanoyl group.
[30] The method as recited in [26] to [29] above, which comprises administering 0.001 mg to 100 mg of the substance or pharmaceutically acceptable salt thereof.
[31] Use of a substance that acts on a growth hormone secretagogue-receptor or a pharmaceutically acceptable salt thereof for producing a spinal neuron damage treating agent.
[32] The use of [31] above, wherein the substance is a peptide selected from the group consisting of:
   (1) a peptide whose amino acid sequence is represented by any one of SEQ ID NO:1 to SEQ ID NO:21, provided that the third amino acid residue from the amino terminus is a modified amino acid residue having a fatty acid introduced in the side chain of that amino acid residue;
   (2) a peptide whose amino acid sequence is represented by any one of SEQ ID NO:1 to SEQ ID NO:21, provided that one or several amino acids in the sequence of from the 5th up to the 28th amino acid from the amino terminus are deleted, substituted and/or added and that the third amino acid residue from the amino terminus is a modified amino acid residue having a fatty acid introduced in the side chain of that amino acid residue, the peptide having a spinal neuronal precursor cell proliferating action; and
   (3) a peptide having a sequence of up to at least the 4th amino acid from the amino terminus of the amino acid sequence that is represented by any one of SEQ ID NO:1 to SEQ ID NO:21, with the third amino acid residue from the amino terminus being a modified amino acid residue having a fatty acid introduced in the side chain of that amino acid residue, the peptide having a spinal neuronal precursor cell proliferating action,
   or a derivative thereof.
[33] The use of [32] above, wherein the substance is a peptide whose amino acid sequence is represented by SEQ ID NO:1, provided that the serine residue which is positioned the third from the amino terminus is a modified amino acid residue having a fatty acid introduced in the hydroxyl group at the side chain of that residue.
[34] The use of [33] above, wherein the substance is a peptide whose amino acid sequence is represented by SEQ ID NO:1, provided that the hydroxyl group at the side chain of the serine residue which is positioned the third from the amino terminus is acylated by an n-octanoyl group.
[35] The use as recited in [31] to [34] above, wherein the spinal neuron damage treating agent contains 0.001 mg to 100 mg of the substance or pharmaceutically acceptable salt thereof.
[36] Use of a substance that acts on a growth hormone secretagogue-receptor or a pharmaceutically acceptable salt thereof for producing an agent for promoting the regeneration of spinal nerves after transplantation of cultured spinal neuronal precursor cells.
[37] The use of [36] above, wherein the substance is a peptide selected from the group consisting of:
   (1) a peptide whose amino acid sequence is represented by any one of SEQ ID NO:1 to SEQ ID NO:21, provided that the third amino acid residue from the amino terminus is a modified amino acid residue having a fatty acid introduced in the side chain of that amino acid residue;
   (2) a peptide whose amino acid sequence is represented by any one of SEQ ID NO:1 to SEQ ID NO:21, provided that one or several amino acids in the sequence of from the 5th up to the 28th amino acid from the amino terminus are deleted, substituted and/or added and that the third amino acid residue from the amino terminus is a modified amino acid residue having a fatty acid introduced in the side chain of that amino acid residue, the peptide having a spinal neuronal precursor cell proliferating action; and
   (3) a peptide having a sequence of up to at least the 4th amino acid from the amino terminus of the amino acid sequence that is represented by any one of SEQ ID NO:1 to SEQ ID NO:21, with the third amino acid residue from the amino terminus being a modified amino acid residue having a fatty acid introduced in the side chain of that amino acid residue, the peptide having a spinal neuronal precursor cell proliferating action,
   or a derivative thereof.
[38] The use of [37] above, wherein the substance is a peptide whose amino acid sequence is represented by SEQ ID NO:1, provided that the serine residue which is positioned the third from the amino terminus is a modified amino acid residue having a fatty acid introduced in the hydroxyl group at the side chain of that residue.
[39] The use of [38] above, wherein the substance is a peptide whose amino acid sequence is represented by SEQ ID NO:1, provided that the hydroxyl group at the side chain of the serine residue which is positioned the third from the amino terminus is acylated by an n-octanoyl group.
[40] The use as recited in [35] to [39] above, wherein the agent for promoting the regeneration of spinal nerves contains 0.001 mg to 100 mg of the substance or pharmaceutically acceptable salt thereof.

### ADVANTAGES OF THE INVENTION

It has been revealed by the present invention that the substances acting on the growth hormone secretagogue-receptor have the action of proliferating spinal neuronal precursor cells. It is based on that action that the substances acting on the growth hormone secretagogue-receptor may be administered to an individual with damaged spinal nerves, making it possible to treat the spinal nerve damage. In addition, when spinal neuronal precursor cells are cultured, the substance(s) acting on the growth hormone secretagogue-receptor may be added to promote cell proliferation, whereby it becomes possible to expedite the use of the cultured spinal neuronal precursor cells in therapy. In other words, in the case of culturing spinal neuronal precursor cells and grafting them to the damaged site of spinal nerves to thereby repair and treat the spinal nerves, it becomes possible to ensure that the cultured spinal neuronal precursor cells are supplied with greater rapidity to the individual.

Further, the cultured spinal neuronal precursor cells may be directly grafted to the affected area and the substance(s) acting on the growth hormone secretagogue-receptor are then administered to the grafted area, whereby it becomes possible to realize promoted curing. In other words, it becomes possible to promote the regeneration of spinal nerves after the transplantation of cultured spinal neuronal precursor cells.

### BRIEF DESCRIPTION OF THE DRAWINGS

[FIG. 1] FIG. 1 shows the expression of GHS-R1a mRNA in the spinal cord.
[FIG. 2] FIG. 2 shows the presence of GHS-R1a in the spinal cord as visualized by immunostaining; A shows the result of staining with an anti-GHS-R antibody; B shows the result of staining with the anti-GHS-R antibody after treating the tissue with the anti-GHS-R antibody; note that FIGS. 2A' are 2B' are diagrams drawn on the basis of FIGS. 2A and 2B.
[FIG. 3-1] FIG. 3-1 shows the co-presence of Nestin, Map2 and GHS-R1a in spinal cord neurons and spinal neuronal precursor cells as visualized by double immunostaining during cell proliferation; A shows MAP2 and BrdU, B shows Nestin and BrdU, C shows Map2 and GHS-R, and D shows Nestin and GHS-R, with the double stained image for each case being shown as Marge.
[FIG. 3-2] FIG. 3-2 is a set of diagrams A', B', C' and D' that are drawn on the basis of A, B, C and D in FIG. 3-1.
[FIG. 4] FIG. 4 shows the action of ghrelin for promoting the incorporation of BrdU into spinal neuronal precursor cells; the numerals represent the mean ± SEM (*: p<0.05).
[FIG. 5] FIG. 5 shows the action of ghrelin for proliferating spinal neuronal precursor cells; A shows the result of detecting BrdU positive cells when ghrelin was not allowed to act, and B shows the result with ghrelin being allowed to act; note that A' are B' are diagrams drawn on the basis of A and B.

### BEST MODES FOR CARRYING OUT THE INVENTION

The growth hormone secretagogue-receptor (GHS-R) is a receptor to which the growth hormone secretagogue (GHS) binds and this hormone is known to occur in several sub-types such as GHS-R1a and GHS-R1b. Among these sub-types, only GHS-R1a is known to activate signal transduction of the receptor downstream linking to phospholipase C, resulting in an increase of intracellular calcium. The term "growth hormone secretagogue-receptor (GHS-R)" as used herein refers to GHS-R1a unless indicated otherwise.

The presence of GHS-R can be confirmed by techniques known to skilled artisans. For example, as will be described later in Example 1, a technique known to skilled artisans is used to extract RNA from the tissue in which the presence of GHS-R is to be confirmed and cDNA is obtained from the RNA. Using a sense primer (e.g. SEQ ID NO:22) and an anti-sense primer (e.g. SEQ ID NO:23) that are specific to GHS-R, the obtained cDNA is amplified by the PCR technique and then electrophoresed to observe the expression of GHS-R mRNA to thereby confirm the presence of GHS-R.

Alternatively, as will be described later in Example 2, a technique known to skilled artisans is used to prepare a frozen slice from the tissue in which the presence of GHS-R is to be confirmed and immunostaining is performed using an anti-GHS-R antibody, followed by examination under a fluorescence microscope to confirm the presence of GHS-R.

Furthermore, as will be described later in Example 3, double staining for both a marker for a specified cell (say, the neuronal precursor marker Nestin or the neuron marker Map2 (microtubule-associated protein 2)) and GHS-R is performed to see if GHS-R has been expressed in the specified cell; the result helps to understand how substances that act on GHS-R will work. For example, Nestin-positive cells are also co-stained with an antibody against GHS-R. In addition, co-localization of Map2 and GHS-R is observed in neurons.

Hereinafter, judgment as to whether a certain substance acts on the growth hormone secretagogue-receptor or not can be made by referring to various indices such as an increase in the calcium ion concentration in cells and other GHS-R mediated physiological actions that are described in the publications listed before.

Hereinafter, judgment as to whether a peptide "has an activity for increasing the calcium ion concentration in cells" can be made by measuring the intracellular calcium ion concentration using a technique known to skilled artisans. For example, one can use FLPR (Fluorometric Imaging Plate Reader, Molecular Devices Corporation) that utilizes the change in fluorescence intensity from Fluo-4 AM (Molecular Probe, Inc.) that occurs on account of a change in calcium ion concentration. Another technique known to skilled artisans may be used to check to see if the peptide having the intracellular calcium ion concentration increasing activity has the growth hormone secretagogue activity either in vitro or *in vivo.* Take, for example, an in *vitro* case; the peptide is added to neurohypophysial cells that have been confirmed not only to secrete the growth hormone but also to express GHS-R and the growth hormone being secreted into the cell culture broth is measured by radioimmunoassay using an anti-growth hormone antibody. In order to confirm the *in vivo* growth hormone secretion stimulating activity, the peptide having the intracellular calcium ion concentration increasing activity may be injected into a peripheral vein in an animal and the growth hormone concentration in the serum is subsequently measured.

Hereinafter, the identity of "a substance having the action for proliferating spinal neuronal precursor cells" can be examined using techniques known to skilled artisans; for example, as will be described later in Examples 4 and 5, using bromodeoxyuridine (BrdU) which is an analogue of thymidine that can be incorporated into DNA specifically for the S phase of the cell cycle, cells are cultured in a BrdU-supplemented medium in the presence or absence of the test substance so that BrdU is incorporated into the cells and, thereafter, an anti-BrdU antibody is used to stain those cells which have incorporated BrdU.

In this case, as mentioned above, double staining may be performed for both a marker for a specified cell (say, the neuronal precursor marker Nestin or the neuron marker Map2) or a specified receptor (say, GHS-R) and BrdU. If more of the cells cultured in the presence of the test substance are found to have incorporated stained BrdU than the cells (control) cultured in the absence of the test substance, the test substance of interest may be held as one that has the action for proliferating spinal neuronal precursor cells. Any significant difference from the control suffices without particular limitation; however, the number of the cells that were cultured in the presence of the test substance and which incorporated the stained BrdU is preferably at least 105%, more preferably at least 110%, in comparison with the control.

As will be typically described later in Examples 4 and 5, another method of investigation comprises culturing cells in a BrdU-supplemented medium in the presence or absence of the test substance so that they incorporate BrdU and, thereafter, measuring the amount of BrdU incorporation into the cells by the ELISA technique. If the cells cultured in the presence of the test substance are found to have incorporated more BrdU than the cells (control) cultured in the absence of the test substance, the test substance of interest may be held as one that has the action for proliferating spinal neuronal precursor cells. Any significant difference from the control suffices without particular limitation; however, the amount of BrdU that was incorporated into the cells cultured in the presence of the test substance is preferably at least 105%, more preferably at least 110%, in comparison with the control.

The substance thus confirmed to have the action for proliferating spinal neuronal precursor cells may be administered, as will be typically described later in Example 6, into a rat model suffering from a damaged spinal cord in combination with the transplantation of spinal neuronal precursor cells, whereupon it can be confirmed that the substance is useful for the recovery of an individual from the spinal cord injury.

The pharmaceuticals of the present invention can be used as pharmaceuticals for animals (individuals). In the present invention, the "substance that acts on the growth hormone secretagogue-receptor" is a substance (ligand) that acts on the growth hormone secretagogue-receptor; preferably, it is a substance that has such an activity that it binds to GHS-R to thereby increase the calcium ion concentration in cells, preferably a substance that has an action for promoting the incorporation of uridine. The growth hormone secretagogue (GHS) may be mentioned as an example. While known peptides and low-molecular weight compounds may be employed as GHS, ghrelin is particularly preferred.

The "peptides" are compounds in which a number of amino acids are connected together by a peptide bond. Here, the amino acids (which may alternatively be designated as amino acid residues) include not only natural amino acids of the general formula: NH₂-CH(R')-COOH where R' has a naturally occurring substituent, but also their D,L-optical isomers and the like. Some natural amino acids may be replaced by modified amino acids (which may alternatively be designated as modified amino acid residues). The modified amino acids include not only those amino acids of the general formula indicated above in which the substituent R' is further modified and their D,L-otpical isomers but also such nonnatural amino acids that the substituent R' in the general formula indicated above has a variety of substituents bonded thereto with or without an intervening moiety such as an ester, ether, thioester, thioether, amide, carbamide or thiocarbamide. Also included are nonnatural amino acids having the amino group in the amino acid replaced by a lower alkyl group.

Hereinafter, the "peptide derivatives" may include such compounds that at least one amino acid in a peptide is replaced by a non-amino acid compound, such compounds that the amino terminus and/or carboxyl terminus of a peptide is modified (say, compounds with the carboxyl terminus being amidated), and such compounds that at least one amino acid in a peptide is replaced by a non-amino acid compound and, in addition, the amino terminus and/or carboxyl terminus is modified; the above-defined "peptides" and "peptide derivatives" are collectively referred to hereinafter as "peptide compounds."

Hereinafter, the "amino acids" include all kinds of amino acids, as exemplified by L-amino acids, D-amino acids, α-amino acids, β-amino acids, γ-amino acids, natural amino acids, and synthetic amino acids.

Hereinafter, the "modified amino acids" means the above-mentioned amino acids in which any desired group is chemically modified. Particularly preferred are such modified amino acids that the α-carbon in an α-amino acid is chemically modified.

Hereinafter, the "ghrelin" refers to a peptide whose amino acid sequence is represented by any one of SEQ ID NO:1 to SEQ ID NO:21, provided that the hydroxyl group at the side chain of the third amino acid residue from the amino terminus is acylated by a fatty acid. The number of carbon atoms in the fatty acid is desirably 2, 4, 6, 8, 10, 12, 14, 16 or 18, with octanoic acid and decanoic acid or their monoenoic fatty acids or polyenoic fatty acids being more desirable, and octanoic acid (carbon number = 8; octanoyl group) being particularly desirable. For each sequence identification number, those having the fatty acids mentioned in the section of "Background Art" are also preferred.

As noted above, ghrelin that can be used encompasses not only the ghrelin derived from humans but also the ghrelin derived from rat, mouse, porcine, bovine and other animals, as well as derivatives thereof.

For a given individual, ghrelin derived from the species to which the individual belongs is desirably used; for instance, human derived ghrelin is desirably used in humans. The human derived ghrelin may be exemplified by a peptide having SEQ ID NO:1 that consists of 28 amino acids, provided that the hydroxyl group at the side chain of the third serine residue from the amino terminus is acylated by a fatty acid (n-octanoyl group). Peptides that can be used as ghrelin derivatives are those peptides whose amino acid sequences are represented by SEQ ID NO:1 to SEQ ID NO:21, provided that one or several amino acids in the sequence of from the 5th up to the 28th (preferably from the 11th to the 28th) amino acid residue from the amino terminus are substituted, inserted or deleted and that the peptides act on the growth hormone secretagogue-receptor (GHS-R).

To be more specific, peptides that retain a sequence of up to at least the 4th, say, up to at least the 5th, preferably up to the 10th, amino acid residue from the amino terminus of the amino acid sequences that are represented by SEQ ID NO:1 to SEQ ID NO:21 and in which the hydroxyl group in the side chain of the third amino acid residue from the amino terminus is acylated by a fatty acid can advantageously be used as ghrelin derivatives; for instance, one may use ghrelin derivatives that have a sequence of up to at least the 5th amino acid residue from the amino terminus of ghrelin, as illustrated by ghrelin(1-5)-Lys-NH2 (GSS(n-octanoyl)FLK-NH2) and ghrelin(1-7)-Lys-NH2(GSS(n-octanoyl)FLSPK-NH2) that are described in Example 5.

By adding basic amino acids to these derivatives, the ghrelin-like activity (intracellular calcium concentration increasing activity) in GHS-R expressing cells is potentiated or by choosing not to terminate the carboxyl terminus of an amino acid with a carboxylic acid but by amidating it to give a form that mimics a peptide bond, it becomes possible to find a minimum active unit of a shorter amino acid sequence; hence, ghrelin derivatives may optionally have a basic amino acid added to the carboxyl terminus or have an amino acid such as the amide form -Lys-NH2 introduced into it.

Hereinafter, the number of amino acids as referred to in the passage reading that "one or several amino acids are substituted, deleted, inserted and/or added" is not particularly limited as long as the peptide comprising the amino acid sequence of interest or its derivatives have the desired function and it may range from about one to nine or from about one to four. If a large number of amino acids are substituted but by amino acids of similar properties (in electric charge and/or polarity), the desired function would not be lost.

It is desired that the amino acid sequences of peptides or their derivatives have homology of at least 70%, preferably at least 80%, more preferably at least 90%, even more preferably at least 95%, and most preferably at least 97%, in comparison with the natural type of amino acid sequence. This is also true with the ghrelin derived from other animals (SEQ ID NO:2 to SEQ ID NO:21).

Other peptides or their derivatives can be designed by referring to the descriptions in documents such as the aforementioned Patent Document 1. For example, preferred peptides or derivatives thereof include:
a peptide that has any one of the amino acid sequences represented by SEQ ID NO:1 to SEQ ID NO:21, preferably SEQ ID NO:1 to SEQ ID NO: 9, more preferably SEQ ID NO:1, provided that the third amino acid from the amino terminus is a modified amino acid having a fatty acid introduced into the side chain, or derivatives of the peptide;
such a peptide that the second or third amino acid residue from the amino terminus of ghrelin is either a modified amino acid residue that has a saturated or unsaturated alkyl chain with 1 to 35 carbon atoms introduced to the α-carbon in an amino acid via an ester, ether, thioether, thioester, amide, carbamide, thiocarbamide or disulfide bond with or without an intervening alkylene group with 1 to 10 carbon atoms, or a modified amino acid residue that has a saturated or unsaturated alkyl chain with 1 to 35 carbon atoms introduced to the α-carbon in an amino acid, or derivatives of the peptide;
such a peptide that the fatty acid introduced into the side chain of ghrelin is a fatty acid selected from the group consisting of fatty acids having 2, 4, 6, 8, 10, 12, 14, 16 and 18 carbon atoms (preferably octanoic acid and decanoic acid or their monoenoic fatty acids or polyenoic fatty acids), or derivatives thereof;
such a peptide that an acidic masking and a basic group are introduced at the carboxyl terminus of ghrelin, or derivatives thereof;
such a peptide that the amino acid at position 3 of ghrelin is a hydrophobic amino acid (e.g., an aromatic hydrophobic amino acid such as tryptophan, cyclohexylalanine or naphthylalanine, or an aliphatic hydrophobic amino acid such as leucine, isoleucine, ylleucine or valine), or derivatives thereof;
such a peptide that the amino acid at position 3 of ghrelin is basic, or derivatives thereof;
such a peptide that the amino acid at position 2 of ghrelin is an amino acid having a comparatively small side chain that will not constrain the degree of freedom of neighboring residues (as exemplified by serine, alanine or norvaline), or derivatives thereof;
such a peptide that the amino acids at positions 3 and 4 of ghrelin are both an L-form, or derivatives thereof; and
such a peptide that the carboxyl terminus of ghrelin or its derivatives is an amide form, or derivatives thereof.

Aside from the foregoing, other substances that act on the growth hormone secretagogue-receptor can be used in the present invention and they include the following peptide compounds, i.e., growth hormone releasing peptide-2 (GHRP-2) (D-Ala-D-BNal-Ala-Trp-D-Phe-Lys-NH₂) and GHRP-6 (His-D-Trp-Ala-Trp-D-Phe-Lys-NH₂) (Muccioli, G et al. : J. Endocrino., 157; 99-106 (1998)), as well as derivatives thereof. Low-molecular weight compounds can also be used and they include L-692,429 (MK-0751) and L-163,191 (MK-0677), etc. (Patchett et al.: Proc. Natl. Acad. Sci., USA, 92, pp. 7001-7005 (1995)).

The substances that act on the growth hormone secretagogue-receptor according to the present invention can be obtained by conventional procedures (see, for example, J. Med. Chem., 43, pp. 4370-4376, 2000, and Patent Document 1). For instance, they can be isolated from naturally occurring raw materials or they can be produced by the recombinant DNA technology and/or chemical synthesis. In addition, if peptide compounds such as ghrelin and its derivatives need to be modified (acylated) in amino acid residues, a modification reaction can be applied in accordance with known means. For example, in a production process using the recombinant DNA technology, host cells transformed with an expression vector having DNA that encodes the peptide compound according to the present invention are cultured and the intended peptide compound is harvested from the culture, whereby the peptide compound according to the present invention can also be obtained. By selecting the host cells, a compound can be obtained such that the intended peptide compound has been modified (say, acylated) in the cultured cells. If the peptide compound has not been modified, a modification reaction such as acylation may optionally be performed in accordance with a known means.

Vectors into which a gene is to be incorporated include *E*. *coli* vectors (e.g., pBR322, pUC18, and pUC19), *B*. *subtilis* vectors (e.g., pUB110, pTP5 and pC194), yeast vectors (e.g., YEp type, YRp type and YIp type), as well as animal cell vectors (e.g., retrovirus and vaccinia virus); any other vectors can be used as long as they can retain the intended gene stably within host cells. These vectors are introduced into suitable host cells. Methods that can be utilized to incorporate the intended gene into plasmids and to introduce them into host cells are exemplified by the methods described in *Molecular Cloning* (Sambrook et al., 1989).

In order to express the intended peptide gene in the above-mentioned plasmids, promoters are connected upstream of that gene in a functional manner.

Any promoters can be used in the present invention as long as they are appropriate for the host cell that is used to express the intended gene. For instance, if the host to be transformed is of the genus *Escherichia,* an lac promoter, a trp promoter, an lpp promoter, a λPL promoter, an recA promoter and the like can be used; in the case of the genus *Bacillus,* an SPO1 promoter, an SPO2 promoter and the like can be used; in the case of yeasts, a GAP promoter, a PHO5 promoter, an ADH promoter and the like can be used; in the case of animal cells, an SV40-derived promoter, a retrovirus-derived promoter and the like can be used.

The thus obtained vectors harboring the intended gene are used to transform the host cells. Exemplary host cells that can be used are bacteria (e.g., of the genus *Escherichia* and the genus *Bacillus*), yeasts (e.g., of the genus *Saccharomyces,* the genus *Pichia*, and the genus *Candida*), and animal cells (e.g., CHO cell and COS cell). Liquid media are suitable for use in culture and it is particularly preferred that they contain a carbon source, a nitrogen source and other nutrients that are necessary for the growth of the transformed cells that are to be cultured. If desired, vitamins, growth secretagogues, sera and the like may optionally be added.

To directly produce fatty acid modified (acylated) peptide compounds, cells are desired that have processing protease activity by which precursor polypeptides for those peptide compounds can be cleaved at suitable positions and that also have an activity by which the serine residue in those peptide compounds can be acylated. Host cells having such processing protease activity and serine acylating activity can be selected by transforming host cells with an expression vector that encodes the precursor polypeptide and confirming that the transformed cells produce a fatty acid modified peptide having a calcium elevating activity or a growth hormone secretion stimulating activity.

After the cultivation, the peptide compound according to the present invention is separated and purified from the culture by conventional procedures. For example, in order to extract the intended substance from the cultured fungus bodies or cells, the fungus bodies or cells are collected after the cultivation and then suspended in a buffer solution containing a protein denaturing agent (e.g., guanidine hydrochloride) and after they are sonicated or otherwise disrupted, the fungus bodies or cells are centrifuged. Subsequently, the intended substance is purified from the supernatant and this can be achieved by suitably combining separating and purifying methods such as gel filtration, ultrafiltration, dialysis, SDS-PAGE and various chromatographic techniques in consideration of the molecular weight of the intended substance, its solubility, electric charge (isoelectric point), affinity, and the like.

The substances that act on the growth hormone secretagogue-receptor according to the present invention (e.g., ghrelin and its derivatives) can be chemically synthesized by conventional techniques. For example, an amino acid having protective groups attached thereto is condensed by the liquid-phase method and/or the solid-phase method to extend the peptide chain and all protective groups are removed with an acid, the resulting crude product being then purified by the above-mentioned methods of purification to obtain the intended substance. An acylating enzyme or an acyl group trnasferase may be used to ensure that the side chain of an amino acid at the intended position is selectively acylated.

A variety of methods are already known for producing peptide compounds and the peptide compounds as the substances according to the present invention can also be produced easily in accordance with known methods; for example, classical methods of peptide synthesis may be complied with or easy production can also be achieved in accordance with the solid-phase method.

If desired, a method in which the recombinant DNA technology is combined with chemical synthesis may be employed to produce peptide compounds; a fragment containing modified amino acid residues is produced by chemical synthesis and another fragment that does not contain any modified amino acid residues is produced using the recombinant DNA technology, with the respective fragments being then fused (see Patent Document 1).

Preferred materials that can be used in the present invention as salts of the substances that act on the growth hormone secretagogue-receptor (e.g., ghrelin and its derivatives) are pharmaceutically acceptable salts and they include, for example, salts with inorganic bases, salts with organic bases, salts with inorganic acids, salts with organic acids, and salts with basic or acidic amino acids.

Advantageous examples of salts with inorganic bases include but are not limited to: alkali metal salts such as sodium salts and potassium salts; alkaline earth metal salts such as calcium salts and magnesium salts; as well as aluminum salts and ammonium salts.

Advantageous examples of salts with organic bases include, but are not limited to, salts with trimethylamine, triethylamine, pyridine, picoline, ethanolamine, diethanolamine, triethanolamine, dicyclohexylamine, and N,N'-dibenzylethylenediamine.

Advantageous examples of salts with inorganic acids include, but are not limited to, salts with hydrochloric acid, hydrobromic acid, nitric acid, sulfuric acid, and phosphoric acid.

Advantageous examples of salts with organic acids include, but are not limited to, salts with formic acid, acetic acid, trifluoroacetic acid, fumaric acid, oxalic acid, tartaric acid, maleic acid, citric acid, succinic acid, malic acid, methanesulfonic acid, benzenesulfonic acid, and p-toluenesulfonic acid.

Advantageous examples of salts with basic amino acids include, but are not limited to, salts with arginine, lysine, and ornithine, and advantageous examples of salts with acidic amino acids include, but are not limited to, salts with aspartic acid and glutamic acid.

Among the salts listed above, sodium salts and potassium salts in particular are most preferred.

The substances that act on the growth hormone secretagogue-receptor according to the present invention (e.g., ghrelin) have been found to possess the action for proliferating spinal neuronal precursor cells as will be shown specifically hereinafter in the Examples and it is particularly noted that they can achieve a significant increase in the number of spinal neuronal precursor cells in embryos. Such cell proliferating action can occur with or without mediation by the growth hormone secretagogue-receptor.

The proliferation of cells can be investigated by techniques known to skilled artisans; for example, it can be investigated by measuring the amount of BrdU incorporated or by counting the number of cells into which BrdU has been incorporated. Such measurement or counting may be performed using the methods described above or the methods that will be described later in the Examples.

The substances that act on the growth hormone secretagogue-receptor according to the present invention (e.g., ghrelin) or pharmaceutically acceptable salts thereof can be used as an active ingredient in a spinal neuron damage treating agent for use in the treatment of spinal neuron damage, an agent for promoting the proliferation of cultured spinal neuronal precursor cells in the culture of spinal neuronal precursor cells, and an agent as well as a therapeutic for promoting the regeneration of spinal nerves after transplantation of cultured spinal neuronal precursor cells.

In addition, by administering the substances that act on the growth hormone secretagogue-receptor according to the present invention (e.g., ghrelin) or pharmaceutically acceptable salts thereof, they can be used in a method for treating spinal neuron damage in an individual, a method for promoting the proliferation of cultured spinal neuronal precursor cells in the culture of spinal neuronal precursor cells, and a method for promoting the regeneration of spinal nerves after transplantation of cultured spinal neuronal precursor cells in an individual.

Furthermore, the substances that act on the growth hormone secretagogue-receptor according to the present invention (e.g., ghrelin) or pharmaceutically acceptable salts thereof can be used to produce a spinal neuron damage treating agent for use in the treatment of spinal neuron damage, an agent for promoting the proliferation of cultured spinal neuronal precursor cells in the culture of spinal neuronal precursor cells, and an agent for promoting the regeneration of spinal nerves after transplantation of cultured spinal neuronal precursor cells.

The drugs of the present invention that contain the substances that act on the growth hormone secretagogue-receptor (e.g., ghrelin) or pharmaceutically acceptable salts thereof as an active ingredient may be mixed with pharmacologically acceptable carriers, excipients, extenders and the like for use in individuals (e.g. human, mouse, rat, rabbit, canine, feline, bovine, equine, porcine, and monkey).

The drugs of the present invention (treating agent and promoting agent) are preferably administered to an individual under regenerative therapy for spinal nerves parenterally, as by intravenous, subcutaneous or intramuscular injection, in a single dose or divided portions of the required quantity; if the individual is a human adult and particularly in the case where he or she is under treatment at home, intranasal administration, pulmonary administration or suppository administration is desirable.

In the present invention, the dosage of the drug is not particularly limited and can be chosen as appropriate for various factors such as the purpose of its use, the age of the individual to which it is to be administered, their body weight, the kind of the individual, the severity of the disease, the state of nutrition, and the drug to be combined for use; if it is to be administered to a human adult in a single dose or divided portions, the substance that acts on the growth hormone secretagogue-receptor (e.g., ghrelin) or a pharmaceutically acceptable salt thereof is preferably contained as an active ingredient in amounts ranging from 0.001 mg to 100 mg, more desirably from 0.01 mg to 10 mg.

As for the period of administration, the above-mentioned dosage is preferably administered once to several times daily for 4 to 24 weeks, more preferably for 4 to 12 weeks.

Pharmaceutically acceptable carriers that can be used are a variety of organic or inorganic carrier substances that are commonly used as pharmaceutical necessities; they are incorporated as an excipient, a lubricant, a binder and a disintegrant in solid preparations or as a solvent, a solubilizing agent, a suspending agent, a tonicity agent, a buffer, a soothing agent, etc. in liquid preparations.

If necessary, pharmaceutical additives may be used, as exemplified by preservatives, antioxidants, coloring agents, and sweeteners.

Advantageous examples of excipients include lactose, sucrose, D-mannitol, starch, microcrystalline cellulose, light silicic anhydride, etc.

Advantageous examples of lubricants include magnesium stearate, calcium stearate, talc, colloidal silica, etc.

Advantageous examples of binders include microcrystalline cellulose, sucrose, D-mannitol, dextrin, hydroxypropyl cellulose, hydroxypropyl methylcellulose, polyvinylpyrrolidone, etc.

Advantageous examples of disintegrants include starch, carboxymethylcellulose, carboxymethylcellulose calcium, croscarmelose sodium, carboxymethylstarch sodium, etc.

Advantageous examples of solvents include water for injection, alcohols, propylene glycol, macrogol, sesame oil, corn oil, etc.

Advantageous examples of solubilizing agents include polyethylene glycol, propylene glycol, D-mannitol, benzyl benzoate, ethanol, trisaminomethane, cholesterol, triethanolamine, sodium carbonate, sodium citrate, etc.

Advantageous examples of suspending agents include: surface active agents such as stearyl triethanolamine, sodium lauryl sulfate, lauryl aminopropionic acid, lecithin, benzalkonium chloride, benzethonium chloride, glyceryl monostearate, etc.; and hydrophilic polymers such as polyvinyl alcohol, polyvinylpyrrolidone, carboxymethylcellulose sodium, methylcellulose, hydroxymethyl cellulose, hydroxyethyl cellulose, hydroxypropyl cellulose, etc.

Advantageous examples of tonicity agents include sodium chloride, glycerin, D-mannitol, etc.

Advantageous examples of buffers include buffer solutions of phosphates, acetates, carbonates, citrates, etc.

Advantageous examples of soothing agents include benzyl alcohol, etc.

Advantageous examples of preservatives include paraoxybenzoic acid esters, chlorobutanol, benzyl alcohol, phenethyl alcohol, dehydroacetic acid, sorbic acid, etc.

Advantageous examples of antioxidants include sulfurous acid salts, ascorbic acid, etc.

The pharmaceutical preparations of the present invention preferably assume dosage forms that are suitable for parenteral administration; dosage forms suitable for parenteral administration include, for example, injections for intravenous, intracutaneous, subcutaneous, intramuscular or other administration, as well as drops, suppositories, transdermal drug delivery systems, transmucosal drug delivery systems, and inhalants; the above-mentioned injections are a preferred dosage form and particularly in the case where the individual is a human adult who is under treatment at home, transmucosal drug delivery systems, inhalants, suppositories and the like are also preferred as dosage forms. These dosage forms are variously known to skilled artisans, who may appropriately select dosage forms that are suitable for the desired route of administration and, if necessary, may use one or two or more pharmaceutical additives available in the art to produce preparations in the form of a pharmaceutical composition.

For example, pharmaceuticals in the form of an injection or a drop can be prepared and provided by the following procedure: the active ingredient, or the substance that acts on GHS-R1a (e.g., ghrelin), is dissolved in distilled water for injection together with one or two or more suitable pharmaceutical additives such as a buffer solution, a sugar solution, a tonicity agent, a pH modifier, a soothing agent and a preservative, subjecting the solution to sterilizing filtration (by passage through a filter), and filling ampoules or vials with the sterilized solution; alternatively, the solution subjected to sterilizing filtration is freeze-dried to formulate a lyophilized preparation. Exemplary additives that can be used include: sugars such as glucose, mannitol, xylitol and lactose; hydrophilic polymers such as polyethylene glycol; alcohols such as glycerol; amino acids such as glycine; proteins such as serum albumin; salts such as NaCl and sodium citrate; acids such as acetic acid, tartaric acid, and ascorbic acid; surface active agents such as Tween 80; and reducing agents such as sodium sulfite. These preparations can be used as an injection or a drop that is prepared by dissolving them in distilled water for injection, physiological saline or the like that are added just before use. For transmucosal administration, intranasal delivery systems (transnasal delivery systems) such as a nasal drop and an intranasal spray are also advantageous, and for transpulmonary administration, an inhalant or the like is also advantageous.

The content of the substance that acts on the growth hormone secretagogue-receptor (e.g., ghrelin) or a pharmaceutically acceptable salt thereof in one preparation ranges from 0.001 mg to 100 mg, preferably from 0.01 mg to 10 mg, and the frequency of administration is desirably from once to several times a day.

In one method of treating the isolated spinal neuronal precursor cells, they are first incubated in a culture broth and to the incubating broth, the substance that acts on the growth hormone secretagogue-receptor (e.g., ghrelin), as prepared by sterilizing filtration or autoclaving sterilization, or a pharmaceutically acceptable salt thereof is added in an amount ranging from 0.1 nM to 1 µM, preferably from 1 nM to 100 nM. The treatment can also be realized by adding 0.0000001 mg/L to 0.1 mg/L of the substance that acts on the growth hormone secretagogue-receptor (e.g., ghrelin) or a pharmaceutically acceptable salt thereof. As will be shown in Examples 4 and 5, this treatment enables promoting the proliferation of spinal neuronal precursor cells that are inherently very slow to proliferate.

Furthermore, as will be described in Example 6, it becomes possible to promote the recovery of an individual from dysfunction due to spinal cord injury.

Hereinafter, any reference to the quantity that is made about the substance (e.g., a peptide compound) or its salt (for example, "containing 0.001 mg to 100 mg of the substance or a pharmaceutically acceptable salt thereof" or "the content of the substance or its salt ranges from 0.0000001 mg/L to 0.1 mg/L") indicates, unless otherwise noted, the amount of that substance itself (e.g., a peptide compound). In other words, unless noted otherwise, the quantity of the salt is indicated as an equivalent amount of the corresponding substance (e.g., a peptide compound).

### EXAMPLES

On the following pages, the present invention is shown specifically by reference to Examples.

### Example 1: Expression of GHS-R1a mRNA in Fetal Rat Spinal Cords

The spinal cord tissue was extracted both from the embryos of pregnant Wistar rats at days 13 to 19 and from rat embryos just after birth and, using the TRIZOL reagent (Life Technologies, Inc., Gaithersburg, MD USA), total RNA was extracted by the method described in Nakahara et al.: Biochem. Biophys. Res. Common. 303: 751 - 755 (2003). From 1 µg of the total RNA, single-strand cDNA was synthesized by random primer reverse transcription using Superscript 3 preamplification reagents (Life Technologies, Inc.) Using a sense primer and an anti-sense primer that were specific for GHS-R1a, the obtained cDNA was amplified by the PCR procedure (using BD Advantage^{™} 2 PCR Enzyme System, BD Science, CA USA) and electrophoresed on a 2% agarose gel. Note that GAPDH (glyceraldehyde 3-phasphate dehydrogenase) featuring stable expression in cells was used as a control mRNA.

The PCR primers specific for GHS-R1a were:
5'-GATACCTCTTTTCCAAGTCCTTCGAGCC-3' (SEQ ID NO:22) for sense; and
5'-TTGAACACTGCCACCCGGTACTTCT-3' (SEQ ID NO:23) for antisense (nucleotides 842-869 and 1001-1025 in accession No. AB001982, GenBank).

The primers specific for GAPDH were:
5'-CGGCAAGTTCAACGGCACA-3' (SEQ ID NO:24) for sense;
5'-AGACGCCAGTAGACTCCACGACA-3' (SEQ ID NO:25) for antisense (nucleotides 1002-1020 and 1125-1147 in accession No. AF106860, GenBank).

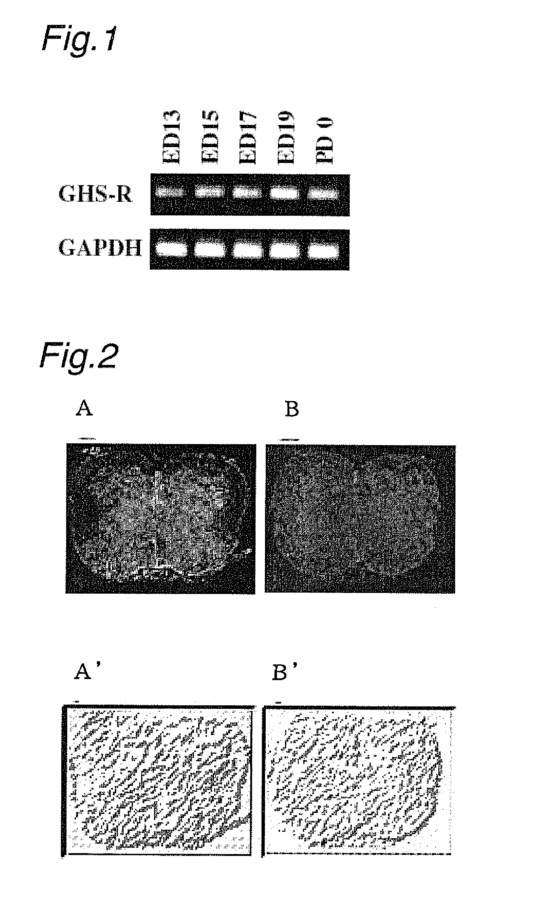

The results are shown in FIG. 1, from which the expression of GHS-R1a mRNA was confirmed in the spinal cords of the embryos from the pregnant rats at days 13, 15, 17 and 19 (ED 13, 15, 17 and 19) and in the spinal cords of the neonatal rats just after birth (PD 0).

### Example 2: Presence of GHS-R1a in Spinal Cord Cells

Fetal spinal cords were collected from a pregnant Wistar rat at day 17 and frozen slices 14 µm thick were prepared. These slices were fixed with 4% paraformaldehyde in 0.1 M phosphate buffer solution for 30 minutes; after washing with 0.1 M phosphate buffer solution, the slices were incubated using 2% normal goat serum in PBS for 30 minutes at room temperature. Thereafter, the slices were washed with PBS three times, incubated with a rabbit anti-GHS-R antibody overnight at 4°C, washed with PBS, and then incubated with Alexa Fluoro 488-conjugated goat anti-rabbit IgG for immunostaining. The residual antibodies were washed out and the slices were embedded for observation under a fluorescence microscope.

The results are shown in FIG. 2. The immunostaining using the anti-GHS-R antibody revealed the presence of GHS-R1a in the gray matter where neuronal cell bodies occurred (FIG. 2A). The intensity of immunostaining dropped greatly as the result of preliminary treatment with the anti-GHS-R antibody (FIG. 2B).

### Example 3: Co-presence of Nestin or Map2 and GHS-R1a in Spinal Neurons and Spinal Neuronal Precursor Cells During Cell Proliferation

Double immunostaining was conducted in order to confirm the co-presence of the neuronal precursor cell marker Nestin or the neutron marker Map2 and GHS-R in proliferating cells (cells that were incorporating BrdU).

Embryos were extracted from a pregnant Wistar rat at day 17 by opening the abdomen under anesthesia. Spinal cords were collected from these embryos and subjected to papain digestion in Hank's balanced salt solution; as a result of subsequent mechanical separation by pipetting, a dispersion of fetal spinal cord cells was obtained. After being filtered and centrifuged, the dispersed cells were suspended in a DMEM medium containing NaHCP₃, 5% fetal bovine serum, penicillin (100 U/mL) and streptomycin (100 µg/mL), followed by plating onto laminin-coated 96-well multi-plates at 10⁵ cells per well.

To the plates, 5-bromo-2'-deoxyuridine (BrdU) (10 µM) was added and incubation was conducted for 4 days to thereby incorporate the BrdU into the spinal cord cells. The spinal cord cells were fixed with methanol and glacial acetic acid at -20 °C for 20 minutes. After DNA denaturation with 2 M HCl, 2% normal goat serum in PBS was used to perform blocking at room temperature for 30 minutes.

To stain for Nestin, an anti-Nestin mouse monoclonal antibody (1:1000, Chemicon International, Inc. CA, USA) was used, and to stain for Map2, a mouse anti-Map2 polyclonal antibody (1:1000, Chemicon International, Inc.) was used; in either case, incubation was conducted overnight at 4°C. Thereafter, in both cases of Nestin and Map2, incubation was conducted at room temperature for one hour using FITC-conjugated goat anti-mouse IgG (1:200, Chemicon International) as a secondary antibody.

After the step of washing the cells, double staining for BrdU was performed using a rat anti-BrdU monoclonal antibody
(1:1000, Abcam, Cambridge, UK) as a primary antibody, and also using Cy^{™} 3-conjugated donkey anti-rat IgG polyclonal antibody
(1:1000, Jackson ImmunoResearch Laboratories, Inc., PA, USA) as a secondary antibody. To perform double staining for GHS-R, the washed cells were fixed with 4% paraformaldehyde in 0.1 M phosphate buffer solution and incubation was first conducted using the above-mentioned anti-Nestin or anti-Map2 antibody, then with a rabbit anti-GHS-R antibody.

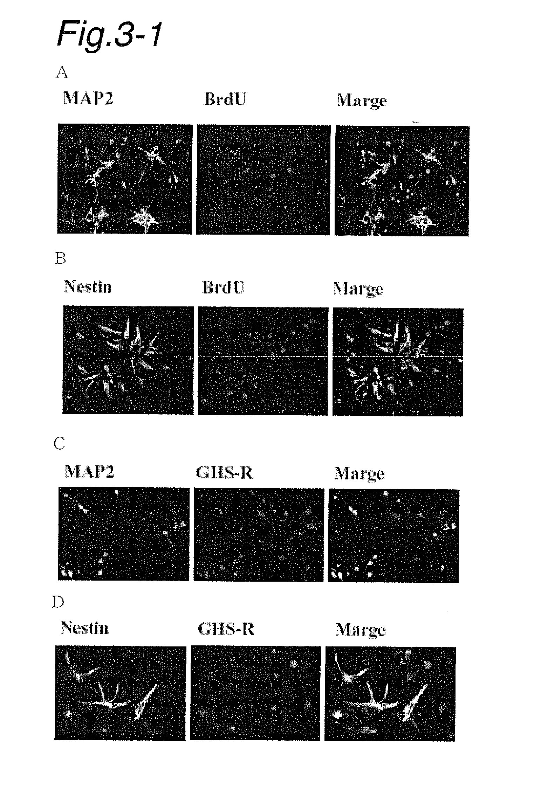

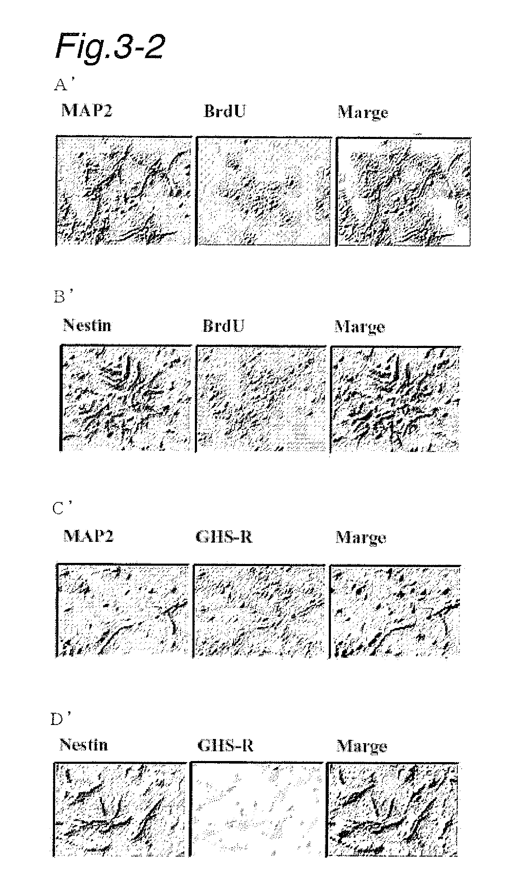

The results are shown in FIG. 3-1. Since GHS-R was also expressed in the dendrite-forming characteristic spinal neurons where Map2 was expressed (FIG. 3-1 C), there was suggested the possibility that ghrelin and other substances acting on GHS-R 1a might contribute to proliferating the spinal neurons. However, since GHS-R was also stained in the cytoplasm of the cells stained for Nestin (FIG. 3-1 D), GHS-R was expressed in spinal neuronal precursor cells, strongly suggesting the possibility that ghrelin and other substances acting on GHS-R 1a would contribute to proliferating the spinal neuronal precursor cells.

Further, looking at the double stained image of the cells stained for Map2 and the BrdU-stained cells after the treatment with ghrelin, the two had not been stained at the same time, which indicates that the cells for which ghrelin shows the proliferating action are not spinal neurons (FIG. 3-1 A). On the other hand, the cells stained for Nestin showed pleomorphism and BrdU had been incorporated into their nuclei, which indicates that those cells were spinal neuronal precursor cells, thus making it clear that ghrelin has an action for proliferating spinal neuronal precursor cells (FIG. 3-1 B).

### Example 4: Ghrelin's Action for Promoting BrdU Incorporation into Cultured Spinal Neuronal Precursor Cells and for Proliferating Spinal Neuronal Precursor Cells

Embryos were extracted from a pregnant Wistar rat at day 17 by opening the abdomen under anesthesia. Spinal cords were collected from these embryos and subjected to papain digestion in Hank's balanced salt solution; as a result of subsequent mechanical separation by pipetting, a dispersion of embryonic spinal cord cells was obtained. After being filtered and centrifuged, the dispersed cells were suspended in a DMEM medium containing NaHCO₃, 5% fetal bovine serum, penicillin (100 U/mL) and streptomycin (100 µg/mL), followed by plating onto laminin-coated 96-well multi-plates at 10⁵ cells per well. After incubation for 4 days, 5-bromo-2'-deoxyuridine (BrdU) (10 µM) was added and incubation was conducted for 6 hours, followed by addition of rat ghrelin (0.003 - 300 nM) and subsequent incubation for 12 hours.

After the end of incubation, the cells were recovered and using Cell Proliferation ELISA Kit (Roche Diagnostic GmbH, Mannheim, Germany), the amount of BrdU incorporation into the cells was measured to investigate the action of ghrelin on BrdU incorporation.

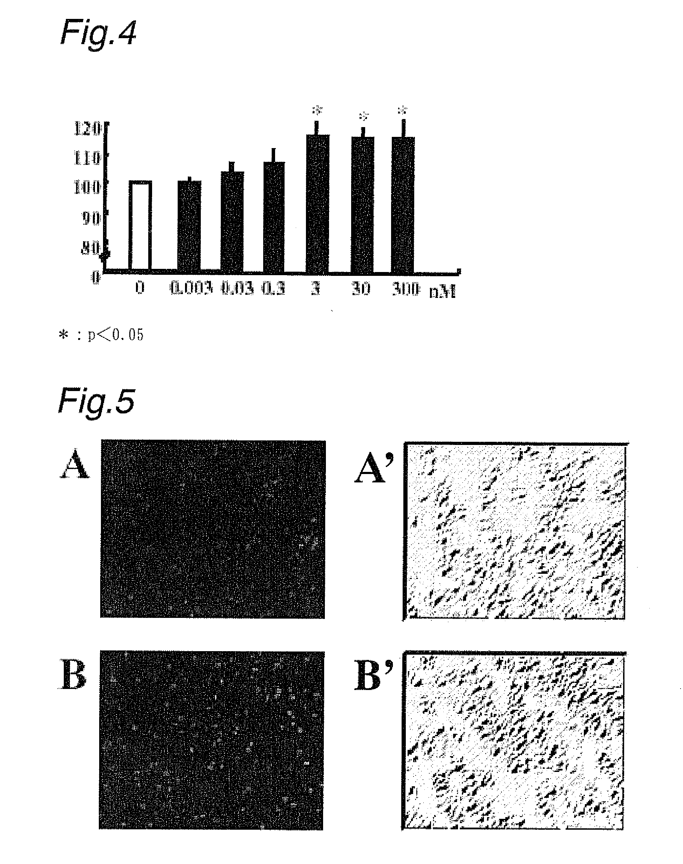

The results are shown in FIG. 4. When ghrelin was allowed to act on the spinal neuronal precursor cells, 3 nM and more of ghrelin could increase the BrdU incorporation into the cells (FIG. 4).

After the end of incubation, the cells were fixed with methanol and glacial acetic acid, had their DNA denatured with 2 N HCl; thereafter, the BrdU incorporated into the cells was measured by detecting the BrdU positive cells using a rat anti-BrdU monoclonal antibody (1:1000, Abcam, Cambridge, UK) as a primary antibody, and also using Cy^{™} 3-conjugated donkey anti-rat IgG polyclonal antibody (1:1000, Jackson ImmunoResearch Laboratories, Inc., PA, USA) as a secondary antibody, in order to investigate the action of ghrelin for proliferating spinal precursor cells.

Comparison was made with a control in which physiological saline was allowed to act instead of ghrelin.

The results are shown in FIG. 5. Compared to the case where ghrelin was not allowed to act (FIG. 5A), a large number of BrdU positive cells were detected when ghrelin was allowed to act (FIG. 5B); thus, it was confirmed at the cellular level that ghrelin has an action for proliferating spinal neuronal precursor cells.

### Example 5: Action of Ghrelin and Derivatives Thereof for_Promoting_BrdU Incorporation into Cultured Spinal Neuronal Precursor Cells

Embryos were extracted from a pregnant Wistar rat at day 16 by opening the abdomen under anesthesia. Spinal cords were collected from these embryos and subjected to papain digestion in Hank's balanced salt solution; as a result of subsequent mechanical separation by pipetting, a dispersion of embryonic spinal cord cells was obtained. After being filtered and centrifuged, the dispersed cells were suspended in a DMEM medium containing NaHCO₃, 5% fetal bovine serum, penicillin (100 U/mL) and streptomycin (100 µg/mL), followed by plating onto laminin-coated 96-well multi-plates at 10⁵ cells per well. After incubation for 4 days, 5-bromo-2'-deoxyuridine (BrdU) (10 µM) was added and incubation was conducted for 6 hours; further, rat ghrelin, ghrelin(1-5)-Lys-NH2 (GSS(n-octanoyl)FLK-NH2), ghrelin(1-7)-Lys-NH2 (GSS(n-octanoyl)FLSPK-NH2) or MK-0677 having the structure indicated below was added in amounts of 0.03 - 3 nM and incubation was conducted for 12 hours before investigating the action of ghrelin on BrdU incorporation.

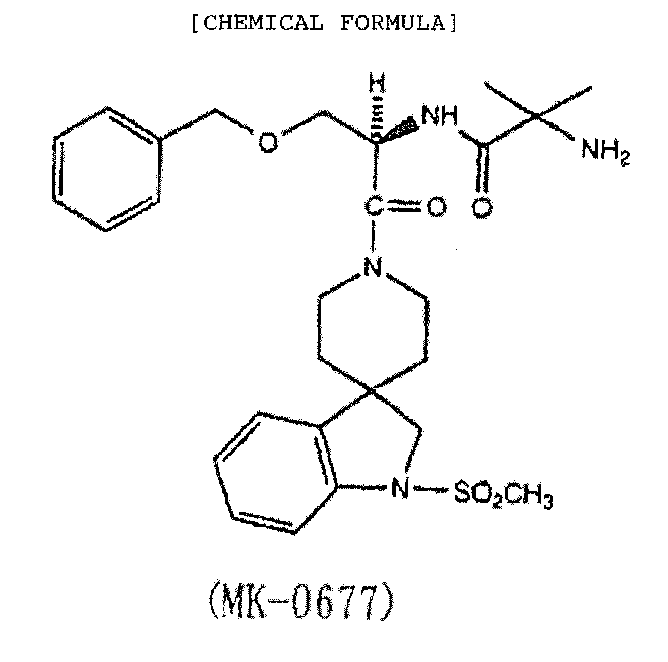

After the end of incubation, the cells were recovered and using Cell Proliferation ELISA Kit (Roche Diagnostic GmbH, Mannheim, Germany), the amount of BrdU incorporation into the cells was measured to investigate the action of ghrelin on BrdU incorporation.

The results are shown in Table 1. When ghrelin was allowed to act on spinal neuronal precursor cells, the BrdU incorporation into the cells was increased by 0.03 to 3 nM or more of ghrelin or 0.3 to 3 nM of ghrelin(1-5)-Lys-NH2 or ghrelin(1-7)-Lys-NH2, thus confirming that ghrelin or derivatives thereof have the action of proliferating fetal spinal neuronal precursor cells.

In addition, it was confirmed at the cellular level that a low enough concentration (0.03 nM) of MK-0677, a chemically synthesized low-molecular weight agonist of GHS-R, has the action of proliferating spinal neuronal precursor cells, as do ghrelin or derivatives thereof.

**[Table 1]**

| Action of Ghrelin and Derivatives Thereof for Promoting BrdU incorporation into Cultured Spinal Neuronal | | | | |
|---|---|---|---|---|
| GHS-R agonist | concentration (nM) | n | Mean ± S.D. | t-test |
| Ghrelin | 0.03 | 6 | 112.5 ± 8.7 | p <0.01 |
| | 0.3 | 6 | 112.0 ± 15.0 | NSD |
| Ghrelin | 3 | 6 | 120.4 ± 7.9 | p <0.01 |
| | | | | |
| Ghrelin (1-5)-LYS-NH₂ | 0.03 | 6 | 106.9 ± 13.2 | NSD |
| | 0.3 | 6 | 114.2 ± 11.3 | p <0.05 |
| | 3 | 6 | 103.5 ± 9.6 | NSD |
| | | | | |
| Ghrelin (1-7)-Lys-NH₂ | 0.03 | 6 | 108.3 ± 11.6 | NSD |
| | 0.3 | 6 | 115.9 ± 14.1 | p <0.05 |
| | 3 | 6 | 140.5 ± 41.9 | p <0.05 |
| | | | | |
| MK-0677 | 0.03 | 6 | 119.5 ± 4.6 | p <0.01 |
| | 0.3 | 6 | 112.0 ± 13.9 | NSD |
| | 3 | 6 | 105.8 ± 13.0 | NSD |
| | | | | |
| Control | 0 | 12 | 100.0 ± 6.7 | - |

| | | | | |
|---|---|---|---|---|
| NSD: No significant difference | | | | |

As shown above, when ghrelin or derivatives thereof were allowed to act on the cultured fetal spinal neuronal precursor cells, more of the BrdU was incorporated into the cells and, at the same time, the number of BrdU positive cells increased; thus, it became clear that ghrelin or derivatives thereof have the action of proliferating spinal neuronal precursor cells.

### Example 6: Assessment of Grafting Spinal Neuronal Precursor Cells and Locally Administering Ghrelin to Rat Models with Injured Spinal Cord

In accordance with the procedure described in Morino, T. et al, Neuroscience Research, 2003, vol. 46, pp 309-318, the grafting of spinal neuronal precursor cells and local administration of ghrelin to rat models with injured spinal cord were assessed. Male SD rats (8 to 9 weeks old) were anesthetized with pentobarbital and incised in the back to expose a vertebra (L11). After excising the vertebral arch, a dental drill was used to open a window with a diameter of about 3 cm, and a stainless steel needle with a silicone rubber fixed thereto was applied vertically, followed by loading of the top with a 20-g weight. Following a compression time of 30 minutes, a damaging drop equivalent to a weight of about 100 g was finally applied from above to construct models suffering a movement disorder in the lower limbs. The animals were divided into three groups: 1) a group under sham operation; 2) a control group with injured spinal cord; and 3) a group with injured spinal cord, treated by cell grafting, and locally administered with ghrelin. The group under sham operation received laminectomy but was simply sutured in the muscle and skin thereafter. After injuring the spinal cord, cultured spinal neuronal precursor cells (10⁵ cells/25 µL) were grafted subdurally. Rat ghrelin was administered subdurally in an amount of 1 nmol together with the cells.

Twenty-four hours after the operation, the rats were transferred into a see-through cage and observed for the frequency of their standing up (by 3-minute observation). More specifically, the frequency of the rat standing up (in such a posture that it lifted the upper limbs and supported the body weight only with the lower limbs) was counted to assess the function of its hind limbs. The results are shown in Table 2.

**[Table 2]**

| Assessment of Grafting Spinal Neuronal Precursor Cells and Locally Administering Ghrelin to Rat Models with Injured Spinal Cord | | | | |
|---|---|---|---|---|
| Group | Initial | 24 hours after operation | t-test* | t-test** |
| Group under sham operation | 10.7 ± 3.8 | 4.3 ± 4.2 | - | - |
| Control group with injured spinal cord | 10.7 ± 2.5 | 1.3 ± 0.6 | NS | - |
| Group with injured spinal Cord, grafted with cells, and administered with ghrelin | 11.7 ± 2.3 | 8.7 ± 4.2 | NS | p<0.05 |

| | | | | |
|---|---|---|---|---|
| The numerals indicate the frequency of a rat standing up per unit time. Note*: Group under sham operation (initial) vs group with injured spinal cord (initial) Note**: Control group with injured spinal cord (after operation) vs group with injured spinal cord and treated (after operation) NS: Not significant | | | | |

As Table 2 shows, the frequency of standing up in the control group for models with injured spinal cord decreased from the initial 10.7 times to 1.3 times; however, the combination of cell grafting and local administration of ghrelin led to a recovery in the frequency of standing up.

## Claims

1. A spinal neuron damage treating agent containing a substance that acts on a growth hormone secretagogue-receptor or a pharmaceutically acceptable salt thereof as an active ingredient.

2. The treating agent of claim 1, wherein the substance is a peptide selected from the group consisting of:
(1) a peptide whose amino acid sequence is represented by any one of SEQ ID NO:1 to SEQ ID NO:21, provided that the third amino acid residue from the amino terminus is a modified amino acid residue having a fatty acid introduced in the side chain of that amino acid residue;
(2) a peptide whose amino acid sequence is represented by any one of SEQ ID NO:1 to SEQ ID NO:21, provided that one or several amino acids in the sequence of from the 5th up to the 28th amino acid residue from the amino terminus are deleted, substituted and/or added and that the third amino acid residue from the amino terminus is a modified amino acid residue having a fatty acid introduced in the side chain of that amino acid residue, the peptide having a spinal neuronal precursor cell proliferating action; and
(3) a peptide having a sequence of up to at least the 4th amino acid residue from the amino terminus of the amino acid sequence that is represented by any one of SEQ ID NO:1 to SEQ ID NO:21, with the third amino acid residue from the amino terminus being a modified amino acid residue having a fatty acid introduced in the side chain of that amino acid residue, the peptide having a spinal neuronal precursor cell proliferating action,
or a derivative thereof.

3. The treating agent of claim 2, wherein the substance is a peptide whose amino acid sequence is represented by SEQ ID NO:1, provided that the serine residue which is positioned the third from the amino terminus is a modified amino acid residue having a fatty acid introduced in the hydroxyl group at the side chain of that residue.

4. The treating agent of claim 3, wherein the substance is a peptide whose amino acid sequence is represented by SEQ ID NO:1, provided that the hydroxyl group at the side chain of the serine residue which is positioned the third from the amino terminus is acylated by an n-octanoyl group.

5. The treating agent as recited in any one of claims 1 to 4, which contains 0.001 mg to 100 mg of the substance or pharmaceutically acceptable salt thereof.

6. An agent for promoting the proliferation of spinal neuronal precursor cells in the culture of spinal neuronal precursor cells that contains a substance that acts on a growth hormone secretagogue-receptor or a salt thereof as an active ingredient.

7. The promoting agent of claim 6, wherein the substance is a peptide selected from the group consisting of:
(1) a peptide whose amino acid sequence is represented by any one of SEQ ID NO:1 to SEQ ID NO:21, provided that the third amino acid residue from the amino terminus is a modified amino acid residue having a fatty acid introduced in the side chain of that amino acid residue;
(2) a peptide whose amino acid sequence is represented by any one of SEQ ID NO:1 to SEQ ID NO:21, provided that one or several amino acids in the sequence of from the 5th up to the 28th amino acid residue from the amino terminus are deleted, substituted and/or added and that the third amino acid residue from the amino terminus is a modified amino acid residue having a fatty acid introduced in the side chain of that amino acid residue, the peptide having a spinal neuronal precursor cell proliferating action; and
(3) a peptide having a sequence of up to at least the 4th amino acid residue from the amino terminus of the amino acid sequence that is represented by any one of SEQ ID NO:1 to SEQ ID NO:21, with the third amino acid residue from the amino terminus being a modified amino acid residue having a fatty acid introduced in the side chain of that amino acid residue, the peptide having a spinal neuronal precursor cell proliferating action,
or a derivative thereof.

8. The promoting agent of claim 7, wherein the substance is a peptide whose amino acid sequence is represented by SEQ ID NO:1, provided that the serine residue which is positioned the third from the amino terminus is a modified amino acid residue having a fatty acid introduced in the hydroxyl group at the side chain of that residue.

9. The promoting agent of claim 8, wherein the substance is a peptide whose amino acid sequence is represented by SEQ ID NO:1, provided that the hydroxyl group at the side chain of the serine residue which is positioned the third from the amino terminus is acylated by an n-octanoyl group.

10. The promoting agent as recited in any one of claims 6 to 9, wherein the content of the substance or salt thereof in the culture medium for spinal neuronal precursor cells is from 0.0000001 mg/L to 0.1 mg/L.

11. An agent for promoting the regeneration of spinal nerves after transplantation of cultured spinal neuronal precursor cells that contains a substance that acts on a growth hormone secretagogue-receptor or a pharmaceutically acceptable salt thereof as an active ingredient.

12. The promoting agent of claim 11, wherein the substance is a peptide selected from the group consisting of:
(1) a peptide whose amino acid sequence is represented by any one of SEQ ID NO:1 to SEQ ID NO:21, provided that the third amino acid residue from the amino terminus is a modified amino acid residue having a fatty acid introduced in the side chain of that amino acid residue;
(2) a peptide whose amino acid sequence is represented by any one of SEQ ID NO:1 to SEQ ID NO:21, provided that one or several amino acids in the sequence of from the 5th up to the 28th amino acid residue from the amino terminus are deleted, substituted and/or added and that the third amino acid residue from the amino terminus is a modified amino acid residue having a fatty acid introduced in the side chain of that amino acid residue, the peptide having a spinal neuronal precursor cell proliferating action; and
(3) a peptide having a sequence of up to at least the 4th amino acid residue from the amino terminus of the amino acid sequence that is represented by any one of SEQ ID NO:1 to SEQ ID NO:21, with the third amino acid residue from the amino terminus being a modified amino acid residue having a fatty acid introduced in the side chain of that amino acid residue, the peptide having a spinal neuronal precursor cell proliferating action
or a derivative thereof.

13. The promoting agent of claim 12, wherein the substance is a peptide whose amino acid sequence is represented by SEQ ID NO:1, provided that the serine residue which is positioned the third from the amino terminus is a modified amino acid residue having a fatty acid introduced in the hydroxyl group at the side chain of that residue.

14. The promoting agent of claim 13, wherein the substance is a peptide whose amino acid sequence is represented by SEQ ID NO:1, provided that the hydroxyl group at the side chain of the serine residue which is positioned the third from the amino terminus is acylated by an n-octanoyl group.

15. The promoting agent as recited in any one of claims 11 to 14, which contains 0.001 mg to 100 mg of the substance or pharmaceutically acceptable salt thereof.

16. A method for promoting the proliferation of cultured spinal neuronal precursor cells, **characterized by** using a substance that acts on a growth hormone secretagogue-receptor or a salt thereof.

17. The method of claim 16, wherein the substance is a peptide selected from the group consisting of:
(1) a peptide whose amino acid sequence is represented by any one of SEQ ID NO:1 to SEQ ID NO:21, provided that the third amino acid residue from the amino terminus is a modified amino acid residue having a fatty acid introduced in the side chain of that amino acid residue;
(2) a peptide whose amino acid sequence is represented by any one of SEQ ID NO:1 to SEQ ID NO:21, provided that one or several amino acids in the sequence of from the 5th up to the 28th amino acid residue from the amino terminus are deleted, substituted and/or added and that the third amino acid residue from the amino terminus is a modified amino acid residue having a fatty acid introduced in the side chain of that amino acid residue, the peptide having a spinal neuronal precursor cell proliferating action; and
(3) a peptide having a sequence of up to at least the 4th amino acid residue from the amino terminus of the amino acid sequence that is represented by any one of SEQ ID NO:1 to SEQ ID NO:21, with the third amino acid residue from the amino terminus being a modified amino acid residue having a fatty acid introduced in the side chain of that amino acid residue, the peptide having a spinal neuronal precursor cell proliferating action,
or a derivative thereof.

18. The method of claim 17, wherein the substance is a peptide whose amino acid sequence is represented by SEQ ID NO:1, provided that the serine residue which is positioned the third from the amino terminus is a modified amino acid residue having a fatty acid introduced in the hydroxyl group at the side chain of that residue.

19. The method of claim 18, wherein the substance is a peptide whose amino acid sequence is represented by SEQ ID NO:1, provided that the hydroxyl group at the side chain of the serine residue which is positioned the third from the amino terminus is acylated by an n-octanoyl group.

20. The method as recited in any one of claims 16 to 19, wherein the content of the substance or salt thereof in the culture medium for spinal neuronal precursor cells is from 0.0000001 mg/L to 0.1 mg/L.
